(19) Europäisches Patentamt
European Patent Office
Office européen des brevets

(11) Veröffentlichungsnummer: **0 286 028 B1**

(12) # EUROPÄISCHE PATENTSCHRIFT

(45) Veröffentlichungstag der Patentschrift: **28.06.95**

(51) Int. Cl.$^{6}$: **C07H 19/16**, C07D 405/04, A61K 31/70, A61K 31/52

(21) Anmeldenummer: **88105277.3**

(22) Anmeldetag: **31.03.88**

(54) **Desaza-purin-nucleosid-Derivate, Verfahren zu deren Herstellung sowie deren Verwendung bei der Nucleinsäure-Sequenzierung sowie als antivirale Mittel.**

(30) Priorität: **10.04.87 DE 3712280**
**20.11.87 DE 3739366**

(43) Veröffentlichungstag der Anmeldung:
**12.10.88 Patentblatt 88/41**

(45) Bekanntmachung des Hinweises auf die Patenterteilung:
**28.06.95 Patentblatt 95/26**

(84) Benannte Vertragsstaaten:
**AT BE CH DE ES FR GB GR IT LI LU NL SE**

(56) Entgegenhaltungen:
**EP-A- 0 251 786**

**JOURNAL OF ORGANIC CHEMISTRY, Band 38, December 1973, Seiten 3179-3186, Ohio,US; T.C. JAIN et al.: "Reactions of 2-Acyloxyisobutyryl Halides withNucleosides. III. Reactions of Tubercidin and Formycin"**

(73) Patentinhaber: **BOEHRINGER MANNHEIM GMBH**

**D-68298 Mannheim (DE)**

(72) Erfinder: **Seela, Frank, Prof.**
**Dahler Heide**
**D-4790 Paderborn (DE)**
Erfinder: **Muth, Heinz-Peter**
**Belmer Strasse 73**
**D-4500 Osnabrück (DE)**
Erfinder: **Kaiser, Klaus**
**Liebigstrasse 50**
**D-4500 Osnabrück (DE)**
Erfinder: **Bourgeois, Werner**
**Wilkienskamp 16**
**D-4500 Osnabrück (DE)**
Erfinder: **Mühlegger, Klaus**
**Römerstrasse 7**
**D-8121 Polling (DE)**
Erfinder: **von der Eltz, Herbert, Dr. rer. nat.**
**In der Au 21**
**D-8120 Weilheim (DE)**
Erfinder: **Batz, Hans-Georg, Dr. rer. nat.**
**Traubinger Strasse 63**
**D-8132 Tutzing (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 286 028 B1

J. AM. CHEM. SOC., Band 106, Oktober 1984, Seiten 6379-6382, Ohio, US; Z.KAZIMIERCZUK et al.: "Synthesis of 2'-Deoxytubercidin, 2'-Deoxyadenosine, andRelated 2'-Deoxynucleosides via a Novel Direct Stereospecific Sodium SaltGlycosylation Procedure"

J. HET. CHEM., Band 23, Februar 1986, Tampa, US; P.K. GUPTA et al.: "Synthesisof beta-D-Ribo and 2'-Deoxy-beta-D-ribofuranosyl Derivatives of 6-Aminopyrazolo[4,3-c]pyridine-4(5H)-one by a Ring Closure of Pyrazole NucleosidePrecursors"

NUCLEIC ACID RES., Band 15, Februar 1987, Seiten 1217-1226, Washington, D.C.,US; H.B. COTTAM et al.: "2'-deoxy-3,7-dideazagunanosine and related compounds.Synthesis of 6-amino-1-(2-deoxy-beta-D-erythro-pentofuranosyl) and 1-beta-D-arabinofuranosyl-1H-pyrrolo[3,2-c]pyridin-4(5H)-one via direct glycosylation ofpyrrole precursor"

CHEMICAL ABSTRACTS, Band 108, 1988, 23. Mai 1988, Seite 748, ZusammenfassungNr. 187138q, Columbus, Ohio, US; F. SEELA et al.: "2'-Deoxy-3,7-dideazanebularine and 2'deoxy-3,7-dideazainosine: synthesis of pyrrolo[3,2-c]pyridine beta-D-2'-deoxyribofuranosides by solid-liquid phase-transfer glycosylation", & HETEROCYCLES 1987, 26(7), 1755-60

**Beschreibung**

Die Erfindung betrifft Desaza-purin-nucleosid-Derivate, Verfahren zur Herstellung dieser Verbindungen sowie die Verwendung dieser Nucleosid-Derivate bei der Sequenzierung von Nucleinsäuren sowie als antivirale Mittel.

Gegenstand der vorliegenden Erfindung sind neue Desaza-purin-nucleosid-Derivateder allgemeinen Formel I

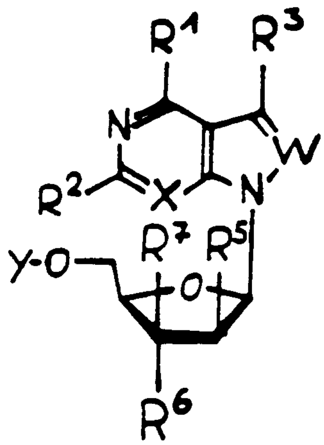

(I)

in der

| | |
|---|---|
| X | Stickstoff oder eine Methingruppe, |
| W | Stickstoff oder die Gruppe |

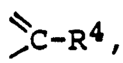

| | |
|---|---|
| $R^1$, $R^2$, $R^3$, $R^4$, | die gleich oder verschieden sein können, Wasserstoff, Halogen, eine Niederalkyl-, Hydroxy-, Mercapto-, Niederalkylthio-, Niederalkyloxy-, Aralkyl-, Aralkyloxy-, Aryloxy- oder eine gegebenenfalls ein- oder zweifach substituierte Aminogruppe, |
| $R^5$ | Wasserstoff oder eine Hydroxygruppe, |
| $R^6$, $R^7$ | jeweils Wasserstoff oder einer der beiden Reste $R^6$ und $R^7$ Halogen, eine Cyano-, eine Azido- oder eine gegebenenfalls ein-oder zweifach substituierte Aminogruppe bedeuten, |

wobei einer der Reste $R^6$ und $R^7$ auch eine Hydroxygruppe vorstellen kann, wenn X eine Methingruppe bedeutet,

und außerdem $R^5$ und $R^7$ zusammen eine weitere Bindung zwischen C-2' und C-3' darstellen können und

Y Wasserstoff, eine Monophosphat-, Diphosphat- oder Triphosphatgruppe vorstellt, mit Ausnahme der Verbindungen 2',3'-Didesoxy-tubercidin, 4,6-Dichloro-1-(2-desoxy-β-D-erythro-pentofuranosyl)pyrrolo[3,2-c]pyridin, 6-Amino-1-(2-desoxy-β-D-erythro-pentofuranosyl)-pyrazolo[4,3-c]pyridin-4(5H)-on, 6-Amino-1-(2-desoxy-β-D-erythro-pentofuranosyl)-1H-pyrrolo[3,2-c]pyridin-4-(5H)-on, 6-Amino-1-β-D-arabinofuranosyl-1H-pyrrolo[3,2-c]pyridin-4(5H)-on, 7-Iodo-2',3'-didesoxy-7-desazaguanosin, 2',3'-Didesoxy-2',3'-didehydro-7-desazaadenosin, 7-Iodo-2',3'-didesoxy-7-desazaadenosin, 7-Iodo-2',3'-didesoxy-7-desazainosin,

sowie mögliche Tautomere und Salze und Nucleinsäuren, die Verbindungen der Formel I als Baustein enthalten.

Die Niederalkylreste in der Definition der Substituenten $R^1$, $R^2$, $R^3$ und $R^4$ können gesättigt oder ungesättigt, geradkettig oder verzweigt sein und 1 - 7, vorzugsweise 1 - 4 Kohlenstoffatome enthalten. Diese Definition der Alkylreste gilt auch für die Alkylreste, die in den Definitionen der Niederalkylthio- und Niederalkoxyreste vorkommen. Ganz besonders bevorzugt sind die Methyl- und die Ethylgruppe.

Unter Halogen in der Definition der Substituenten $R^1$, $R^2$, $R^3$, $R^4$, $R^6$ und $R^7$ werden Fluor, Chlor, Brom und Jod verstanden.

Die in den Definitionen der Substituenten $R^1$, $R^2$, $R^3$ und $R^4$ vorkommenden Aralkyl- bzw. Aralkoxy-Reste enthalten einen Alkylrest mit 1 bis 5, vorzugsweise 1 - 3 Kohlenstoffatomen, die ein- oder mehrfach mit einem aromatischen Rest, beispielsweise Phenyl- oder Naphthylrest, substituiert sind. Die aromatischen Reste können ihrerseits ein- oder mehrfach durch eine Alkyl- oder Alkoxygruppe mit jeweils 1 - 3 Kohlenstoffatomen substituiert sein. Besonders bevorzugt ist die Benzylgruppe.

Als Aryloxy-Rest in der Definition der Substituenten $R^1$, $R^2$, $R^3$ und $R^4$ sind besonders Phenyloxy-Reste bevorzugt, die gegebenenfalls ein- oder mehrfach durch weitere Substituenten, wie beispielsweise Nitro-, Alkyl- und Alkoxygruppen substituiert sein können.

Die in der Definition der Substituenten $R^1$, $R^2$, $R^3$, $R^4$, $R^6$ und $R^7$ vorkommende Aminogruppe, die gegebenenfalls ein- oder zweifach substituiert sein kann, enthält als mögliche Substituenten vorzugsweise Alkylgruppen mit 1 - 5, vorzugsweise 1 - 3 Kohlenstoffatomen, die ihrerseits wiederum durch Alkoxygruppen, Halogen oder gegebenenfalls ein- oder mehrfach substituierte Aminogruppen substituiert sein können. Diese Substituenten können auch einen Aralkylrest vorstellen. Die beiden Stickstoffsubstituenten können auch zusammen einen Alkyliden, vorzugsweise einen Methyliden-Rest darstellen, der seinerseits durch Alkoxy, substituierte Aminogruppen oder Halogen substituiert sein kann. Ein ganz bevorzugter Substituent dieser Art ist die Dimethylaminomethyliden-Gruppe.

Die Monophosphatgruppe ist die Gruppe $-PO(OH)_2$, die Diphosphatgruppe, die Gruppe $-P_2O_3(OH)_3$ und die Triphosphatgruppe bedeutet die Gruppe $-P_3O_5(OH)_4$.

Als mögliche Salze kommen vor allem Alkali-, Erdalkali- und Ammoniumsalze der Phosphatgruppen in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1 - 4 Kohlenstoffatome oder/und Aralkylreste, bevorzugt Benzylreste, substituiert sein kann. Die Substituenten können hierbei gleich oder verschieden sein. Die Salze der Phosphate können auf bekannte Weise in die freien Säuren überführt werden.

Die Verbindungen der Formel I können basische Gruppen, insbesondere Amino-Gruppen enthalten, die mit geeigneten Säuren in Säureadditionsalze übergeführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

Die aus der allgemeinen Formel I ausgenommenen Verbindungen sind aus dem Stand der Technik bereits bekannt und in den folgenden Dokumenten beschrieben: J. Org. Chem. 38, Nr. 18 (1973), S. 3179 - 3186; J. Am. Chem. Soc. 106, Nr. 21 (1984), S. 6379-6382; J. Heterocyclic Chem. 23, 59 (1986), S. 59 - 64; Nucleic Acid Res. 15, Nr. 2 (1987), S. 1217 - 1226; EP-A-251786.

Die neuen Verbindungen der allgemeinen Formel I können in Analogie zu bekannten, verwandten Verbindungen hergestellt werden. Als besonders zweckmäßig hat sich zur Herstellung der Verbindungen der Formel I ein Verfahren erwiesen, bei dem man eine Verbindung der Formel II

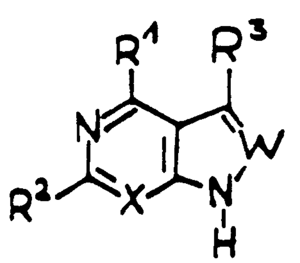

(II)

in der

X, W, $R^1$, $R^2$ und $R^3$ die oben angegebene Bedeutung haben,

mit einer Verbindung der Formel III

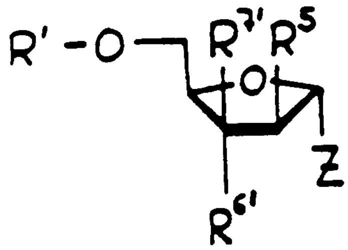

(III)

in der

- $R^5$ die oben angegebene Bedeutung hat,
- $R^{6'}$, $R^{7'}$ jeweils Wasserstoff oder einer der beiden Reste $R^{6'}$ und $R^{7'}$ eine Azido- oder eine durch eine Sauerstoffschutzgruppe geschützte Hydroxygruppe,
- $R'$ eine Sauerstoffschutzgruppe und
- Z eine reaktive Gruppe bedeuten

zu Verbindungen der Formel IV

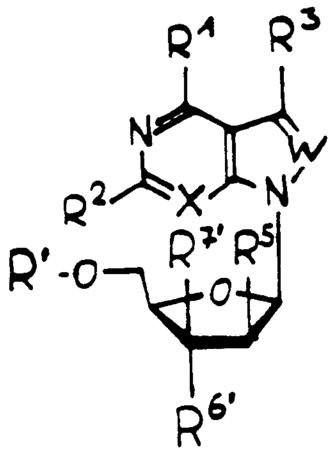

(IV)

in der

X, W, $R^1$, $R^2$, $R^3$, $R^5$, $R^{6'}$, $R^{7'}$ und $R'$ die oben angegebene Bedeutung haben,
umsetzt und gegebenenfalls vorhandene Sauerstoffschutzgruppen abspaltet
und danach gegebenenfalls
eine so erhaltene Verbingung, in der $R^6$ oder $R^7$ eine Hydroxygruppe bedeutet, nach vorherigem selektivem Schutz der 5′-Hydroxygruppe mit einem Halogenid, Cyanid oder Azid in bekannter Weise in eine Verbindung der Formel I, in der $R^6$ und $R^7$ Halogen oder eine Cyano- oder Azidogruppe bedeutet, überführt oder in bekannter Weise zu einer Verbindung der Formel I, in der $R^6$ oder $R^7$ Wasserstoff bedeutet, desoxygeniert
oder eine so erhaltene Verbindung der Formel I, in der $R^6$ oder $R^7$ eine Azidogruppe bedeutet, in bekannter Weise zu einer Verbindung der Formel I, in der $R^6$ oder $R^7$ eine Aminogruppe bedeutet, reduziert
und gewünschtenfalls anschließend Verbindungen der Formel I, in denen Y Wasserstoff bedeutet, in bekannter Weise in die Mono-, Di-oder Triphosphate überführt
und gewünschtenfalls erhaltene freie Basen bzw. Säuren in die entsprechenden Salze oder erhaltene Salze in die entsprechenden freien Basen bzw. Säuren umwandelt.

Die Verbindungen der Formel II werden mit den Verbindungen der Formel III umgesetzt, besonders vorteilhaft unter Phasentransferbedingungen. Unter den Bedingungen der Phasentransferkatalyse werden die Basen der Formel II in ein entsprechendes Anion überführt, beispielsweise durch 50 %ige wässrige Natronlauge. Das so entstandene Anion wird durch einen Phasentransferkatalysator, beispielsweise Tris[2-(2-methoxyethoxy) ethyl]amin, hydrophobiert und in die organische Phase transportiert, in der es mit der reaktiven Verbindung der Formel III abreagiert.

Als reaktive Gruppen Z in den Verbindungen der allgemeinen Formel III kommen vorzugsweise Halogenreste und Alkoxygruppen in Frage. Die Hydroxygruppen des Zuckerrestes werden bei dieser Umsetzung in üblicher Weise durch dem Fachmann geläufige Sauerstoffschutzgruppen, beispielsweise

Toluoyl-, Benzoyl-oder Acetylgruppen, geschützt. Die Sauerstoffschutzgruppen können nach beendeter Umsetzung in bekannter Weise unter alkalischen Bedingungen wieder abgespalten werden, zeckmäßigerweise verwendet man eine 1M methanolische Methanolatlösung.

Es kann zweckmäßig sein, auch die Reste $R^1$, $R^2$, $R^3$ und $R^4$ während der Reaktion durch geeignete Schutzgruppen geschützt zu halten.

Eine weitere vorteilhafte Methode zur Herstellung der Verbindungen der Formel IV stellt das Fest-Flüssig-Phasentransfer-Verfahren unter Verwendung von festem, pulverförmigem Kaliumhyroxid, dem oben genannten Kryptanden, sowie den Verbindungen der Formeln II und III in einem aprotischen Lösungsmittel dar.

Verbindungen der Formel I, in denen $R^6$ oder $R^7$ Halogen oder eine Azidogruppe bedeutet, werden vorzugsweie hergestellt, indem man von Verbindungen der Formel I ausgeht, in der $R^6$ oder $R^7$ eine Hydroxygruppe vorstellt. Die Hydroxygruppe in 5′-Stellung wird zunächst selektiv geschützt. Auch hierzu stehen dem Fachmann bekannte Verfahren zur Verfügung. Beispielsweise hat sich in der Nucleotidchemie die 4,4′-Dimethoxytriphenylmethylgruppe bewährt. Diese kann nach erfolgter Umsetzung wieder leicht durch milde Säurehydrolyse abgespalten werden während die ebenfalls säurelabile glycosidischen Bindung unter diesen Bedingungen nicht hydrolysiert wird. Die Umsetzung des zu schützenden Nucleosids mit dem Sauerstoffschutzgruppenreagenz für die 5′-Hydroxygruppe wird in einem geeigneten organischen Lösungsmittel, zweckmäßigerweise getrocknetem Pyridin, mit einem leichten Überschuß des Sauerstoffschutzgruppenreagenzes sowie gegebenenfalls einer geeigneten Hilfsbase, beispielsweise N-Ethyldiisopropylamin, durchgeführt. Die so geschützte Verbindung der Formel I wird mit einem Halogenid, zweckmäßigerweise einem Alkalihalogenid oder einem organischen Halogenid, bzw. mit einem Azid, zweckmäßigerweise mit einem Alkaliazid, in allgemein bekannter Weise umgesetzt. Die OH-Gruppe am C-3′-Atom wird dabei nucleophil durch das Halogenid bzw. Azid substituiert.

Verbindungen der Formel I, in der $R^6$ oder $R^7$ eine Hydroxygruppe bedeutet, können auch nach vorherigem Schutz der 5′-Hydroxygruppe in vorstehender weise, nach bekannten Methoden desoxygeniert werden, wobei Verbindungen der Formel I entstehen, in denen $R^6$ und $R^7$ Wasserstoff bedeutet. Hierzu wird die Verbindung der allgemeinen Formel I, in der $R^6$ oder $R^7$ eine Hydroxygruppe vorstellt und bei der die 5′-Hydroxygruppe in vorstehender Weise geschützt worden ist und auch sonstige funktionelle Reste Schutzgruppen trage, zunächst in ein 3′-O-Thiocarbonylderivat überführt, welches anschließend mit Tributylzinnhydrid radikalisch reduziert wird. Solche Methoden zur Desoxygenierung von 2′-Desoxynucleosiden zu 2′,3′-Didesoxynucleosiden sind dem Fachmann bekannt. Als besonders günstig hat sich die Methode der Barton-Desoxygenierung erwiesen (J. Chem. Soc., Perkin Trans. I (1975) 1574).

Verbindungen der Formel I, in denen $R^6$ oder $R^7$ eine Aminogruppe bedeutet, werden zweckmäßigerweise hergestellt, indem man Verbindungen der Formel I, in der dieser Rest $R^6$ oder $R^7$ eine Azidogruppe darstellt, reduziert. Diese Reduktion der Azidogruppe zur Aminogruppe kann nach verschiedenen, allgemein bekannten Methoden erfolgen. Besonders vorteilhaft hat sich die Reduktion mit Wasserstoff an einem Palladium-Kohlekatalysator erwiesen.

Die Phosphatgruppen werden in Verbindungen der allgemeinen Formel I, in denen Y Wasserstoff bedeutet, in bekannter Weise eingeführt. Die Monophosphate erhält man beispielsweise, indem man Verbindungen der Formel I, in denen Y Wasserstoff bedeutet, mit Phosphoroxychlorid in Trimethylphosphat phosphoryliert. Die auf diesem Wege erhaltenen Triethylammoniumsalze können in bekannter Weise in andere Salze durch Umsalzen überführt werden. Die Di-bzw. Triphosphate werden nach bekannten Methoden, vorzugsweise aus den Monophosphaten durch Umsetzung mit o-Phosphaten bzw. Pyrophosphaten erhalten. Ihre verschiedenen Salze können ebenfalls nach bekannten Methoden hergestellt werden.

Die Verbindungen der Formel II sind bekannte Verbindungen oder können in Analogie zu bekannten Verbindungen hergestellt werden. Derartige Herstellungsverfahren sind beispielsweise beschrieben in Chemische Berichte 110 (1977) 1462, J. Chem. Soc. 1960, 131 bzw. Tetrahedron Letters 21 (1980) 3135.

Auch die Verbindungen der Formel III sind zum Teil bekannte Verbindungen. Bisher nicht beschriebene Verbindungen können in völliger Analogie zu den bekannten Verbindungen hergestellt werden. Die Herstellung einer solchen Verbindung ist beispielsweise beschrieben in Chem. Ber. 93 (1960) 2777 bzw. Synthesis 1984, 961.

Die neuen Verbindungen der vorliegenden Erfindung weisen wertvolle pharmakologische Eigenschaften auf. Insbesondere wird durch Inhibierung des Enzyms reverse Transkriptase die Vermehrung von Retroviren verhindert, d. h. die erfindungsgemäßen Substanzen haben insbesondere cytostatische sowie antivirale Eigenschaften.

Die Bausteine der Nucleinsäuren enthalten als glycosidische Komponente entweder den β-D-Ribofuranosylrest oder dessen 2′-Desoxy-Derivat. Neben diesen aglykonischen Resten werden in Nucleosid-Antibiotika modifizierte D-Ribofuranosyl-Derivate gefunden. So enthält z. B. Cordycepin, das aus den Kulturfiltraten

von Cordyceps miltairs isoliert werden kann, das Monosaccharid Cordycepose. Neben diesem 2′- bzw. 3′-Desoxy-Derivat der Ribonucleoside hat man vor geraumer Zeit 2′,3′-Didesoxynucleoside synthetisch dargestellt. Sie wirken antiviral und können speziell über die Inhibierung des Enzyms Reverse Transkriptase die Vermehrung von Retroviren hemmen (vergleiche Proc. Natl. Acad. Sci. USA 83 (1986) 1911 bzw. Nature 325 (1987) 773). Von besonderem therapeutischem Interesse ist die Hemmwirkung auf das HIV-Virus, den Verursacher des AIDS. Sie haben allerdings den Nachteil, daß sie Inhibitoren auch der zellulären DNA-Polymerase sind, so daß sie cytotoxisch wirken. Weiterhin können sie durch zelluläre Enzyme deaktiviert werden.

Diese nachteile weisen die Verbindungen gemäß Formel I nicht auf. Sie wirken antivirial, ohne cytotoxisch zu sein.

Die erfindngsgemäßen Substanzen der Formel I lassen sich auch vorteilhaft bei der DNA-Sequenzierung nach Sanger einsetzen. Besonders des Sequenzierung d(G-C)-reicher DNA-Fragmente wird durch die Bildung von Sekundärstrukturen, die zu einer Bandenkompression im Bereich von d(G-C)-Clustern führen, erschwert. Der Grund hierfür leigt in der Hoogsteen-Basenpaarung von Guanosinmolekülen. Durch den Ersatz von 2′-Desoxyguanosintriphosphat durch erfindungsgemäße Verbindungen, in denen $R^6$ eine Hydroxygruppe vorstellt, wird die Bandenkompression größtenteils behoben.

Die erfindungsgemäßen Verbindungen der Formel I, in denen $R^6$ und $R^7$ Wasserstoff bedeuten, werden bei der DNA-Sequenzierung nach Sanger als Kettenterminatoren anstelle der bekannten 2′,3′-Didesoxyverbindungen verwendet.

Nucleinsäuren, die als Baustein eine oder mehrere Verbindungen der Formel I enthalten, können nach bekannten Verfahren hergestellt werden (beispielsweise Nucleic Acids Research Vol. 14, Nr. 5, 1986, S. 2319 ff). Sie entstehen aber auch beispielsweise bei der DNA-Sequenzierung. Werden als Bausteine Verbindungen der Formel I verwendet, in welcher $R^6$ eine Hydroxygrupp bedeutet, so kann eine Nucleinsäure mehrere solcher Bausteine aufweisen; wird als Baustein eine Verbindung der Formel I verwendet, in der $R^6$ Wasserstoff bedeutet, so kann ein solcher Baustein nur einmal, nämlich am Kettenende eingebaut sein. Die erfindungsgemäßen Nucleinsäuren sind aus 2 bis 1000, vorzugsweise 8 bis 50 Nucleotidbausteinen aufgebaut. Besonders bevorzugt sind Nucleinsäuren mit 15 - 30 Nucleotidbausteinen.

Diese Nucleinsäuren können ebenfalls als antivirale Mittel eingesetzt werden. Als sogenannte anti-sense-Nucleinsäure hybridisiert diese Nucleinsäure mit der ssDNA/RNA des Virus und erschwert die Transskription zur Virus-DNA. Solche Nucleinsäuren können insbesondere als Mittel gegen AIDS verwendet werden, da sie nicht oder nur schwer durch zelleigene Restriktionsenzyme abgebaut werden.

Zur Herstellung von Arzneimitteln werden die Substanzen der allgemeinen Formel I, ihre pharmakologisch verträglichen Salze oder sie enthaltende Nucleinsäuren in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Waser oder Öl, wie z. B. Olivenöl, suspendiert oder gelöst.

Die erfindungsgemäßen Substanzen können in flüssiger oder fester Form enteral oder parenteral appliziert werden. Als Injektionsmedium kommt vorzugsweise Wasser zur Anwendung, welches die bei Injektionslösungen üblichen Zusätze, wie Stabilisierungsmittel, Lösungsvermittler oder Puffer enthält.

Derartige Zusätze sind z. B. Tartrat- und Citratpuffer, Ethanol, Komplexbildner (wie Ethylendiamintetraessigsäure und deren nichttoxischen Salze) und hochmolekulare Polymere (wie flüssiges Polyethylenoxid) zur Viskositätsregulierung. Feste Trägerstoffe sind z. B. Stärke, Lactose, Mannit, Methylcellulose, Talkum, hochdisperse Kielselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die erfindungsgemäßen Verbindungen werden üblicherweise in Mengen von 1 - 100 mg, vorzugsweise 2 - 80 mg pro Tag und pro kg Körpergewicht appliziert. Bevorzugt ist es, die Tagesdosis auf 2 -5 Applikationen zu verteilen, wobei bei jeder Applikation 1 - 2 Tabletten mit einem Wirkstoffgehalt von 5 - 1000 mg verabreicht werden. Die Tabletten können auch retardiert sein, wodurch sich die Anzahl der Applikationen pro Tag auf 1 - 3 vermindert. Der Wirkstoffgehalt der retardierten Tabeltten kann 20 - 2000 mg betragen. Der Wirkstoff kann auch durch Injektion ein- bis achtmal pro Tag bzw. durch Dauerinfusion gegeben werden, wobei Mengen von 50 - 4000 mg/Tag normalerweise ausreichen.

Die Erfindung wird durch die folgenden Beispiel näher erläutert.

**Beispiel 1**

2-Amino-7-desaza-2′,3′-didesoxy-9-β-D-ribofuranosyl-purin-6-on

a) 2-[(4,4′-Dimethoxytriphenylmethyl)amino]-7-desaza-2′-desoxy-5′-0-(4,4′-dimethoxytriphenylmethyl)-9-β-D-ribofuranosyl-purin-6-on

1.0 g(3.8 mmol) 7-Desaza-2′-desoxyguanosin wird zweimal mit trockenem Pyridin abgedampft und in 20 ml Pyridin suspendiert. Man fügt 4.0 g (11.8 mmol) 4,4′-Dimethoxytriphenylmethylchlorid und 2.5 ml (14.6 mmol) Hünig-Base (N-Ethyldiisopropylamin) hinzu und rührt 3 Stunden bei Raumtemperatur.

Die Reaktionsmischung wird anschließend in 150 ml 5 %ige, wässerige $NaHCO_3$-Lösung gegeben und zweimal mit je 150 ml $CH_2Cl_2$ extrahiert. Die vereinigten organischen Extrakte weren über $Na_2SO_4$ getrocknet, filtriert und an Kieselgel 60 H (Säule 10 × 4 cm, $CH_2Cl_2$/Aceton (9:1)) chromatographiert. Nach Einengen der Hauptzone wird der Rückstand in wenig $CH_2Cl_2$ gelöst und in eine Mischung aus n-Hexan/Ether (1:1) eingetropft. Nach Filtration erhält man 2.04 g (61 %) der gewünschten farblosen, amorphen Verbindung. - DC (Kielselgel, $CH_2Cl_2$/Aceton (8:2)): $R_F$ = 0.7.- UV (MeOH): $\lambda_{max}$ = 272, 283 nm (Sch.) ($\epsilon$ = 18800, 16400).-
$^1$H-NMR ([$D_6$]DMSO): $\delta$ = 1.75 (m, 2′-$H_b$), 1.86 (m, 2′-$H_a$), 3.09 (m, 5′-H), 3.79 (m, 4′-H), 4.10 (m, 3′-H), 5.19 (d, 3′-OH, J = 4.3 Hz), 5.61 (pt, 1′-H, J = 6.5 Hz), 6.16 (d, 6-H, J = 3.5 Hz), 6.62 (d, 5-H, J = 3.5 Hz), 10.35 (s, NH).

| | | | | |
|---|---|---|---|---|
| $C_{53}H_{50}N_4O_8$ (871.0) | Ber.<br>Gef. | C 73.09<br>C 73.02 | H 5.79<br>H 5.98 | N 6.43<br>N 6.34 |

b) 2-[(4,4′-Dimethoxytriphenylmethyl)amino]-7-desaza-2′-desoxy-3′-O-phenoxythiocarbonyl-5′-O-(4,4′-diemthoxytriphenylmethyl)-9-β-D-ribofuranosyl-purin-6-on

Eine Suspension aus 1.0 g (1.1 mmol) der Verbindung aus 1a) in 15 ml trockenem Acetonitril wird mit 300 mg (2.5 mmol) p-Dimethylaminopyridin und 300 µl (2.2 mmol) Phenoxythiocarbonylchlorid versetzt und 16 Stunden bei Raumtemperatur gerührt. Die Reaktionsmischung wird eingedampft und der Rückstand an Kielselgel 60 H (Säule 10 × 4 cm, $CH_2Cl_2$/Aceton (8:2)) chromatographiert. Der durch Eindampfen der Hauptzone erhaltene Rückstand wird in wenig $CH_2Cl_2$ gelöst und durch Eintropfen in eine Mischung aus n-Hexan/Ether (1:1) ausgefällt, man erhält 0.99 g (89 %) farblose, amorphe Substanz.- DC (Kieselgel, $CH_2Cl_2$/Aceton (8:2)): $R_F$ = 0.8- UV (MeOH): $\lambda_{max}$ = 269, 282 nm (Sch.) ($\epsilon$ = 19300, 16000).-

$^1$H-NMR ([$D_6$]DMSO): $\delta$ = 2.06 (m, 2′-$H_b$), 2.34 (m, 2′-$H_a$), 3.26 (m, 5′-H), 4.25 (m, 4′-H), 5.61 (m, 3′-H und 1′-H), 6.23 (d, 6-H, J = 3.5 Hz), 6.67 (d, 5-H, J = 3.5 Hz), 10.41 (s, NH).

| | | | | | |
|---|---|---|---|---|---|
| $C_{60}H_{54}N_4O_9S$ (1007.2) | Ber.<br>Gef. | C 71.77<br>C 71.26 | H 5.40<br>H 5.43 | N 5.56<br>N 5.52 | S 3.18<br>S 3.11 |

c) 2-[(4,4′-Dimethoxytriphenylmethyl)amino]-7-desaza-2′-3′-didesoxy-5′-O-(4,4′-diemthoxytriphenylmethyl)-9-β-D-ribofuranosyl-purin-6-on

500 mg (0.5 mmol) der Verbindung aus 1b) in 20 ml frisch destilliertem Toluol werden mit 30 mg (0.2 mmol) 2,2′-Azo-bis-(2-methylpropionsäurenitril) und 300 µl (1.1 mmol) Tributylzinnhydrid versetzt und 3 Stunden unter Argon bei 80° C gerührt (DC-Kontrolle, $CHCl_3$/Methanol (97:3). Nach vollständiger Umsetzung wird die Reaktionsmischung eingedampft und der Rückstand an Kieselgel 60 H (Säule 30 × 4 cm, $CHCl_3$/Methanol (99:1)) chromatographiert. Nach Eindampfen der Hauptzone und Aufnehmen in wenig $CH_2Cl_2$ werden 320 mg (75 %) der gewünschten amorphen, farblosen Verbindung durch Eintropfen in n-Hexan/Ether gefällt.- DE (Kieselgel, $CH_2Cl_2$/Methanol (95:5)): $R_F$ = 0.5
$^1$H-NMR ([$D_6$]DMSO): $\delta$ = 1.60, 1.80 (2 m, 2′-H und 3′-H), 3.07 (m, 5′-H), 4.06 (m, 4′-H), 5.43 (m, 1′-H), 6.11 (d, 6-H, J = 3.5 Hz), 6.65 (d, 5-H, J = 3.5 Hz), 10.34 (s, NH).

d) 2-Amino-7-desaza-2′,3′-didesoxy-9-β-D-ribofuranosyl-purin-6-on

300 mg (0.35 mmol) der Verbindung aus 1c) werden in 10 ml 80 %iger Essigsäure gelöst und 15 Minuten bei Raumtemperatur gerührt. Anschließend wird das Lösungsmittel im Ölpumpenvakuum asbgezogen und der Rückstand mehrmals mit Wasser abgedampft. Das Rohprodukt wird an Kieselgel 60 H (Säule 10 × 4 cm, $CH_2Cl_2$/Methanol (9:1)) chromatographiert. Die durch Eindampfen der Hauptfraktion erhaltene, schaumige Substanz wird aus wenig Methanol kristallisiert, man erhält 50 mg (57 %) farblose Nadeln vom Schmp. 228° C (Zers.).- DC (Kieselgel, $CH_2Cl_2$/Methanol (9:1)): $R_F$ = 0.3.
UV (MeOH):$\lambda_{max}$ = 261, 281 nm (Sch.) ($\epsilon$ = 13300, 7800).-
$^1$H-NMR ([$D_6$]DMSO): $\delta$ = 1.96 (m, 3′-H), 2.08, 2.27 (2 m, 2′-$H_a$ und 2′-$H_b$), 3.48 (m, 5′-H), 3.97 (m, 4′-H), 4.86 (t, 5′-OH, J = 5.4 Hz), 6.12 (pt, 1′-H, J = 5.5 Hz), 6.24 (m, $NH_2$ und 6-H), 6.92 (d, 5-H, J = 3.5 Hz), 10.34 (s, NH).

| | | | | |
|---|---|---|---|---|
| $C_{11}H_{14}N_4O_3$ (250.3) | Ber.<br>Gef. | C 52.79<br>C 52.98 | H 5.64<br>H 5.80 | N 22.39<br>N 22.55 |

In analoger Weise erhält man über die entsprechenden 2′-Desoxynucleoside und anschließende Deoxigenierung, wie unter c) beschrieben:

A) 3,7-Didesaza-2′,3′-didesoxy-9-β-ribofuranosyl-purin

UV (0.1 n-HCl): $\lambda_{max}$ = 224, 274 nm

| | | | | |
|---|---|---|---|---|
| $C_{12}H_{14}N_2O_2$ (218.2) | Ber.<br>Gef. | C 66.0<br>C 66.1 | H 6.4<br>H 6.4 | N 12.8<br>N 12.6 |

B) 3,7-Didesaza-2′,3′-didesoxy-9-β-D-ribofuranosyl-purin-6-on

UV (Methanol): $\lambda_{max}$ = 264 nm ($\epsilon$ = 11600), 282 nm ($\epsilon$ = 8000) 295 nm ($\epsilon$ = 5200)

| | | | | |
|---|---|---|---|---|
| $C_{12}H_{14}N_2O_3$ (234.2) | Ber.<br>Gef. | C 61.5<br>C 61.3 | H 6.0<br>H 6.1 | N 11.95<br>N 11.8 |

C) 2-Chlor-6-methoxy-3,7-didesaza-2′,3′-didesoxy-9-β-D-ribofuranosyl-purin

UV (Methanol) $\lambda_{max}$ = 271, 280 nm

| | | | | |
|---|---|---|---|---|
| $C_{13}H_{15}N_2O_3Cl$ (282.6) | Ber.<br>Gef. | C 55.2<br>C 55.1 | H 5.3<br>H 5.3 | N 9.9<br>N 9.9 |

D) 6-Amino-3,7-didesaza-2′,3′-didesoxy-9-β-D-ribofuranosyl-purin

| | | | | |
|---|---|---|---|---|
| $C_{12}H_{15}N_3O_2$ (233.2) | Ber.<br>Gef. | C 63.65<br>C 63.62 | H 6.16<br>H 6.11 | N 17.13<br>N 17.01 |

UV (Methanol) $\lambda_{max}$ 271 nm ($\epsilon$ = 12800)

E) 3,7-Didesaza-2′,3′-didesoxy-9-β-D-ribofuranosyl-purin-2,6-dion

| | | | | |
|---|---|---|---|---|
| $C_{12}H_{14}N_2O_4$ (250.2) | Ber.<br>Gef. | C 57.55<br>C 57.50 | H 5.6<br>H 5.7 | N 11.2<br>N 11.2 |

**Beispiel 2**

2-{[(Dimethylamino)methlen]amino}-7-desaza-2′,3′-didesoxy-9-β-D-ribofuranosyl-purin-6-on

a) 2-{[(Dimethylamino)methylen]amino}-7-desaza-2′-desoxy-9-β-D-ribofuranosyl-purin-6-on

270 mg (1.01 mmol) 7-Desaza-2′-desoxy-guanosin in 5 ml trockenem, aminfreiem Dimethylformamid werden mit 2 ml (11.7 mmol) N,N-Deimthylformamiddiethylacetal versetzt und 1 Stunde bei 50° C unter Argon gerührt. Anschließend wird die Reaktionsmischung im Vakuum eingedampft und an Kieselgel 60 H (Säule 10 × 4 cm, $CH_2Cl_2$/Methanol (9:1)) chromatographiert. Durch Abdampfen des Lösungsmittels erhält man aus der Hauptzone 230 mg (71 %) hellgelbe, amorphe Substanz.-
DC (Kieselgel, $CH_2Cl_2$/Methanol (9:1)): $R_F$ = 0.3.- UV (MeOH): $\lambda_{max}$ = 240, 311 nm (ε = 18300, 17400).-
$^1$H-NMR ([D$_6$]DMSO. δ = 2.15 (m, 2′-H$_b$), 2.41 (m, 2′-H$_a$), 3.02, 3.15 (s, 2 $CH_3$), 3.52 (m, 5′-H), 3.79 (m, 4′-H), 4.32 (m, 3′-H), 4.91 (t, 5′-OH, J = 5.4 Hz), 5.27 (d, 3′-OH, J = 3.5 Hz), 6.34 (d, 6-H, J = 3.5 Hz), 6.45 (pt, 1′-H, J = 7.0 Hz), 7.07 (d, 5-H, J = 3.5 Hz), 8.56 (s, NH=C), 11.04 (s, NH).

| | | | | |
|---|---|---|---|---|
| $C_{14}H_{19}N_5O_4$ (321.3) | Ber.<br>Gef. | C 52.33<br>C 52.48 | H 5.96<br>H 6.14 | N 21.79<br>N 21.69 |

b) 2-{[(Dimethylamino)methylen]amino}-7-desaza-2′-desoxy-5′-O-(4,4′-dimethoxytriphenylmethyl)-9-β-D-riobfuranosyl-purin-6-on

100 mg (0.31 mmol) der Verbindung aus 2a) werden in 2 ml trockenem Pyridin gelöst, mit 170 mg (0.5 mmol) 4,4′-Diemthoxytriphenylmethylchlorid und 0.2 ml (1.2 mmol) Hünig-Base versetzt und 3 Stunden bei Raumtemperatur gerühet. Anschließend wird die Reaktionsmischung eingedampft und der Rückstand an Kieselgel 60 H (Säule 10 × 2.5 cm, Solvens $CHCl_3$/Methanol (99:1)) chromatographiert. Der durch Eindampfen der Hauptfraktion erhaltene Rückstand wird in $CH_2Cl_2$ gelöst und durch Eintropfen in eine Mischung aus n-Hexan/Ether (1:1) werden 160 mg (84 %) farblose, amorphe Substanz gefällt.-
DC (Kieselgel, $CH_2Cl_2$/Methanol (9:1): $R_F$ = 0.6.-
UV (MeOH):$\lambda_{max}$ = 236, 311 nm (ε = 38200, 18100).-
$^1$H-NMR ([D$_6$]DMSO): δ = 2,23 (m, 2′-H$_b$), 2.42 (m, 2′-H$_a$), 3.03 (s, $CH_3$), 3.14 (m, 5′-H und $CH_3$), 3.90 (m, 4′-H), 4.33 (m, 3′-H), 5.34 (d, 3′-OH, J = 4.3 Hz), 6.34 (d, 6-H, J = 3.5 Hz), 6.49 (pt, 1′-H, J = 6.8 Hz), 6.90 (d, 5-H, J = 3.5 Hz), 8.58 (s, NH=C), 11.07 (s, NH).

| | | | | |
|---|---|---|---|---|
| $C_{35}H_{37}N_5O_6$ (623.7) | Ber.<br>Gef. | C 67.40<br>C 67.31 | H 5.98<br>H 6.00 | N 11.23<br>N 11.17 |

c) 2-{[(Dimethylamino)methylen]amino}-7-desaza-2′-desoxy-3′-O-phenoxythiocarbonyl-5′-O-(4,4′-dimethoxytriphenylmethyl)-9-β-D-ribofuranosyl-purin-6-on

900 mg (1.4 mmol) der Verbindung aus 2b), gelöst in 15 ml trockenem $CH_2Cl_2$, werden mit 340 mg (2.8 mmol) p-Di-methylaminopyridin und 250 µl (1.8 mmol) Phenoxythicarbonylchlorid versetzt und 16 Stunden bei Raumtemperatur gerühet. Die Lösung wird im Vakuum eingedampft und der Rückstand an Kieselgel 60 H (Säule 20 × 4 cm, $CHCl_3$/Aceton (7:3), $CHCl_3$/Aceton (6:4)) chromatographiert. Der durch Eindampfen der Hauptzone erhaltene Rückstand wird in wenig $CH_2Cl_2$ auffenommen und durch Eintropfen in n-Hexan/Ether (1:1) werden 980 mg (90 %) der gewünschten farblosen, amorphen Verbindung gefällt.-

DC (Kieselgel, $CH_2/Cl_2$/Methanol (95:5)): $R_F$ = 0.5.- UV (MeOH): $\lambda_{max}$ = 235, 277 (Sch.), 283, 312 nm ($\epsilon$ = 41300, 11400, 12600, 17000).-
$^1$H-NMR ([$D_6$]DMSO): $\delta$ = 2.73 (m, 2′-$H_b$), 2.97 (m, 2′-$H_a$), 3.01, 3.10 (s, 2 $CH_3$), 3.37 (m, 5′-H), 4.33 (m, 4′-H), 5.90 (m, 3′-H), 6.40 (d, 6-H, J = 3.5 Hz), 6.55 (pt, 1′-H)< 6.98 (d, 5-H, J = 3.5 Hz), 8.58 (s, CH=N), 11.30 (s, NH).

| | | | | | |
|---|---|---|---|---|---|
| $C_{42}H_{41}N_5O_7S$ (759.9) | Ber.<br>Gef. | C 66.39<br>C 66.49 | H 5.44<br>H 5.55 | N 9.22<br>N 9.25 | S 4.22<br>S 4.29 |

d) 2-{[(Dimethylamino)methylen]amino}-7-desaza-2′,3′-didesoxy-5′-O-(4,4′-dimethoxytriphenylmethyl)-9-β-D-ribofuranosyl-purin-6-on

500 mg (0.7 mmol) der Verbindung asu 2c), gelöst in 20 ml frisch destilliertem Toluol, werden mit 25 mg (0.15 mmol) 2,2′-Azobis-(2-methylpropionsäurenitril) und 500 µl (1.9 mmol) Tributylzinnhydrid versetzt und bei 80° C unter Argon 16 Stunden gerührt. Anschließend wird die Reaktionsmischung im Ölpumpenvakuum eingedampft und der Rückstand an Kieselgel 60 H (Säule 20 × 4 cm, $CH_2Cl_2$/Aceton (9:1), $CHCl_3$/Aceton (7:3), $CHCl_3$/Aceton (6:4)) chromatographiert. Der durch Einengen der Hauptfraktion erhaltene Rückstand wird in wenig Dichlormethan gelöst und durch Eintropfen in n-Hexan/Ether gefällt, man erhält 320 mg (80 %) der gewünschten farblosen, amorphen Verbindung.
DC (Kieselgel, $CH_2Cl_2$/Methanol (95:5)): $R_F$ = 0.3.-
UV (MeOH): $\lambda_{max}$ = 236, 277 (Sch.), 284, 312 nm ($\epsilon$ = 37200, 12000, 13500, 18000).-
$^1$H-NMR ([$D_6$]DMSO): $\delta$ = 2.02 (m, 3′-H), 2.20, 2.33 (m, 2′-$H_a$ und 2′-$H_b$), 3.02, 3.13 (s, 2 $CH_3$), 3.08 (m, 5′-H), 4.17 (m, 4′-H), 6.31 (d, 6-H, J = 3.5 Hz), 6.38 (m, 1′-H), 6.92 (d, 5-H, J = 3.5 Hz), 8.61 (s, CH=N), 11.03 (s, NH).

| | | | | |
|---|---|---|---|---|
| $C_{35}H_{37}N_5O_7$ (607.7) | Ber.<br>Gef. | C 69.18<br>C 69.23 | H 6.14<br>H 6.24 | N 11.52<br>N 11.61 |

e) 2-{[(Dimethylamino)methylen]amino}-7-desaza-2′,3′-didesoxy-9-β-D-ribofuranosyl-purin-6-on

130 mg (0.21 mmol) der Verbindung aus 2d) werden in 5 ml 80 %iger Essigsäure gelöst und 15 Minuten bei Raumtemperatur gerührt. Anschließend dampft man die Essigsäure im Ölpumpenvakuum ab und chromtographiert den Rückstand an Kieselgel 60 H (Säule 20 × 2 cm, $CH_2Cl_2$/Methanol (95:5)). Der durch Einengen der Hauptfraktion erhaltene Rückstand wird durch wiederholtes Abdampfren mit Aceton aufgeschäumt, man erhält 43 mg (67 %) der gewünschten farblosen, amorphen Verbindung.-
DC (Kieselgel, $CH_2Cl_2$/Methanol (9:1): $R_F$ = 0.5.-
UV (MeOH): $\lambda_{max}$ = 239, 282 (Sch.), 311 nm ($\epsilon$ = 17400, 10500, 16900).-
$^1$H-NMR ([$D_6$]DMSO): $\delta$ = 2.06, 2.32 (m, 2′-H und 3′-H), 3.01, 3.14 (s, 2 $CH_3$), 3.51 (m, 5′-H), 4.00 (m, 4′-H), 4.87 (t, 5′-OH), 6.33 (m, 1′-H und 6-H, J = 3.3 Hz), 7.05 (d, 5-H, J = 3.3 Hz), 8.59 (s, CH=N), 11.02 (s, NH).

| | | | | |
|---|---|---|---|---|
| $C_{14}H_{19}N_5O_3$ (305.3) | Ber.<br>Gef. | C 55.07<br>C 55.23 | H 6.27<br>H 6.41 | N 22.94<br>N 22.75 |

## Beispiel 3

2-Amino-6-methoxy-7-desaza-2′,3′-didesoxy-9-β-D-ribofuranosyl-purin

a) 2-Amino-6-methoxy-7-desaza-2′,desoxy-9-β-D-ribofuranosyl-purin

543 mg (10 mmol) fein gepulvertes Kaliumhydroxid und 68 mg (0.2 mmol) Tetrabutylammoniumhydrogensulfat in 30 ml absolutem Dichlormethan werden 15 Minuten unter $N_2$-Atmosphäre bei Raumtemperatur

gerührt. Anschließend wird mit 330 mg (2 mmol) 2-Amino-6-methoxy-7-desaza-purin (2-Amino-4-methoxy-7H-pyrrolo[2,3-d]pyrimidin) versetzt und weitere 30 Minuten gerührt. Nach Zugabe von 884 mg (2,2 mmol) 2-Desoxy-3,5-di-O-p-toluoyl-β-D-erythro-pentofuranosylchlorid läßt man noch weitere 3 Minuten rühren. Man saugt unlösliche Bestandteile ab, wäscht mit wenig Dichlormethan und engt das Filtrat auf etwa 10 ml ein. Nach Versetzen mit 3 ml 1M-Natriummethoxid in Methanol läßt man 3 Stunden bei Raumtemperatur rühren. Nach Neutralisation mit Essigsäure wird das Lösungsmittel abgezogen, der Rückstand in heißem Wasser aufgenommen, filtriert und das Filtrat an einer Dowex-(1 x 2 OH-Form, 30 x 2 cm)-Ionenaustauschersäule chromatographiert (Wasser/Methanol 9:1). Nach Abziehen des Lösungsmittels und Umkristallisation aus Wasser erhält man aus den Hauptzonen 260 mg (63 %) farblose Kristalle.
Schmp. 152 - 154° C.
DC (Kieselgel, $CH_2Cl_2$/MeOH 9:1) $R_F$ = 0.7
UV (methanol) $\lambda_{max}$ = 225, 259, 285 (ε = 24900, 3600, 7600). $^1$H-NMR ([D$_6$]DMSO): δ = 6.27 (1H, d, J = 3.7 Hz), 6.42 (1H, dd, $J_{1',2'a}$ = 8.4 Hz, $J_{1',2'b}$ = 5.9 Hz), 7.10 (1H, d, J = 3.7 Hz) ppm.
$^{13}$C-NMR ([D$_6$]DMSO): δ = 52.49 ($OCH_3$), 82.37 (C=1′), 98.85 (C-5), 119.45 (C-6) ppm.

b) Die nach a) erhaltene Verbindung 2-Amino-6-methoxy-7-desaza-2′-desoxy-9-β-D-ribofuranosyl-purin wird wie in Beispiel 1c) beschrieben, zu 2-Amino-6-methoxy-7-desaza-2′,3′-didesoxy-9-β-D-ribofuranosyl-purin desoxigeniert.

## Beispiel 4

2-Amino-6-chlor-7-desaza-2′,3′-didesoxy-9-β-D-robofuranosyl-purin

a) Die Verbindung wird nach Acetylierung von 2-Amino-7-desaza-2′,3′-didesoxy-9-β-D-ribofuranosyl-purin-6-on (hergestellt nach Beipiel 1d) nach der in Liebigs Ann. Chem. 1987, 15 - 19 beschriebenen Methode durch Halogenierung hergestellt.
b) Das resultierende Rohgemisch wird zur Entfernung der Acetylschutzgruppe 3 Stunden mit methanolischer Ammoniaklösung bei Raumtemperatur stehengelassen, bis zur Trockne eingeengt und anschließend auf Kieselgel mit dem Laufmittel Chloroform/Methanol chromatographiert. Nach Vereinigen der Hauptfraktionen und Eindampfen wird aus $H_2O$ kristallisiert.
UV (Methanol): $\lambda_{max}$ = 235, 258, 316 (ε = 27800, 4300, 5800)

| | | | | | |
|---|---|---|---|---|---|
| $C_{11}H_{13}N_4O_2Cl$ (268.7): | Ber.<br>Gef. | C 49.1<br>C 49.3 | H 4.8<br>H 4.85 | N 20.8<br>N 20.7 | Cl 13.0<br>Cl 13.1 |

## Beispiel 5

2-Amino-7-desaza-2′,3′-didesoxy-9-β-D-ribofuranosyl-purin

268 mg (1 mmol) 2-Amino-6-chlor-7-desaza-2′,3′-didesoxy-9-β-D-ribofuranosyl-purin werden in 25 ml 70 %igem, wässrigen Methanol gelöst, zu einer Suspension von 30 mg vorhydriertem Pd/C (10 %ig) in 25 ml 70 %igem wässrigem Methanol gegeben und bis zur beendeten $H_2$-Aufnahme hydriert. Man zieht das Lösungsmittel ab und kristallisiert aus Methanol. Ausbeute 180 mg (77 %).

| | | | | |
|---|---|---|---|---|
| $C_{11}H_{14}N_4O_2$ (234,3) | Ber.<br>Gef. | C 56.4<br>C 56.3 | H 6.0<br>H 6.0 | N 23.9<br>N 23.7 |

UV (Methanol): $\lambda_{max}$ = 234, 256, 314 nm (ε = 30.600, 4100, 5200)

## Beispiel 6

2-Amino-6-mercapto-7-desaza-2′,3′-didesoxy-9-β-D-ribofuranosyl-purin

536 mg (2 mmol) 2-Amino-6-chlor-7-desaza-2′,3′-didesoxy-9-β-D-ribofuranosyl-purin und 1,5 g (20 mmol) Thioharnstoff werden in 30 ml Ethanol suspendiert und während ca. 15 Stunden am Rückfluß erhitzt.

Danach destilliert man das Lösungsmittel ab, nimmt den Rückstand in etwa 25 ml Methanol auf und chromtographiert an Kieselgel 60 H (Säule 20 × 3 cm, $CH_2Cl_2$/MeOH 9:1). Durch Eindampfen der Hauptfraktion und Kristallisation aus Methanol/$H_2O$ erhält man 230 mg (43 %) der Thioverbindung.

| | | | | |
|---|---|---|---|---|
| $C_{11}H_{14}N_4O_2S$ (266.3) | Ber.<br>Gef. | C 49.6<br>C 49.4 | H 5.3<br>H 5.4 | N 21.0<br>N 21.1 |

UV (Methanol): $\lambda_{max}$ = 235, 271, 345 nm ($\epsilon$ = 17600, 11700, 18700)
$^1$H-NMR ([$D_6$]DMSO): $\delta$ = 1.9 (m, 3′-H), 2.1 (m, 2′-$H_b$), 2.34 (m, 2′-$H_a$), 3.50 (m, 5′-H), 3.97 (m, 4′-H), 4.86 (t, 5′-OH), 6.12 (m, 1′-H), 6.24 (m, $NH_2$ und 8-H), 6.92 (d, 7-H), 11.1 (s, NH)

**Beispiel 7**

2,6-Diamino-7-desaza-2′,3′-didesoxy-9-$\beta$-D-ribofuranosyl-purin

268 mg (1 mmol) 2-Amino-6-chlor-7-desaza-2′,3′-didesoxy-9-$\beta$-D-ribofuranosyl-purin werden mit 40 ml wässriger, konzentrierter Ammoniaklösung versetzt und 60 Stunden bei 65° C im Wasserbad gut verschlossen erwärmt. Nach Abdampfen des Lösungsmittels wird an einer Kielselgel-Säule chromatographiert, zuerst mit $CH_2Cl_2$/MeOH (9:1) (Ausgangsmaterial), dann mit $CHCl_3$/Methanol (4:1). Nach Kristallisation aus Wasser erhält man 120 mg (48 %) der Diaminoverbindung.,

| | | | | |
|---|---|---|---|---|
| $C_{11}H_{15}N_5O_2$ (249.3) | Ber.<br>Gef. | C 53.0<br>C 53.15 | H 6.0<br>H 5.9 | N 28.1<br>N 28.2 |

UV (Methanol): $\lambda_{max}$ = 264. 284 nm ($\epsilon$ = 9800, 8000) $^1$-H-NMR ([$D_6$]DMSO): $\delta$ = 1.9 (m, 3′-H), 2.1, 2.4 (2 m, 2′-$H_{a,\ b}$),3.4 (m, 5′-H), 3.8 (m, 4′-H), 4.8 (t, 5′-OH), 5.6 (s, $NH_2$), 6.2 (dd, 1′-H), 6.3 (d, 7-H), 6.7 (s, $NH_2$), 6.9 (d, 8-H)

**Beispiel 8**

2-Methylthio-6-methoxy-7-desaza-2′,3′-didesoxy-9-$\beta$-D-ribofuranosyl-purin

a) 2-Methylthio-6-methoxy-7-desaza-2′-desoxy-9-$\beta$-D-ribofuranosyl-purin

500 mg (2.56 mmol) 4-Methoxy-2-methylthio-7H-pyrrolo[2,3-d]-pyrimidinund 400 mg (1.75 mmol) Benzyltriethylammoniumchlorid, gelöst in 20 ml Dichlormethan mit 20 ml 50 %iger Natronlauge als Gegenphase werden mit dem Vibromischer kurz durchgemsicht. Man versetzt mit 1.2 g (3.1 mmol) 2-Desoxy-3,5-di-O-(p-toluoyl)-$\beta$-D-erythro-pentofuranosylchlorid in wenig Dichlormethan und setzt das Vribromischen 30 Minuten fort. Die organische Phase wird abgetrennt und die wässrige mit Dichlormethan gegengeschüttelt. Die vereinigten organischen Extrakte werden mit Wasser gewaschen und mit Natriumsulfat getrocknet. Nach Filtration wird eingedampft und der Rückstand in 100 ml 1 M Natriummethanolat in Methanol gelöst. Man läßt ca. 12 Stunden bei Raumtemperatur rühren, dampft ein, nimmt in Wasser auf und adsorpbiert an einer Dowex 1-X2-Ionenaustauschersäule (30 x 3 cm, $OH^-$-Form. Elution mit Wasser-Methanol (1:1) führt zu einer Hauptzone. Nach dem Verdampfen des Lösungsmittels wird aus Wasser umkristallisiert; Ausbeute 321 mg (40 %) farblose Nadeln mit Schmp. 118° C. - DC (Kieselgel/$CH_2Cl_2$/Aceton (8:2)) $R_F$ = 0.26. - UV (Methanol): $\lambda_{max}$ = 283, 236 nm ($\epsilon$= 13000, 155500). $^1$H-NMR ([$D_6$]DMSO): $\delta$ = 2.20 (m, 2′H), 2.40 (m, 2′-H), 2.56 (s, $CH_3S$), 3.50 (m, 5′-$H_2$), 3.81 (m, 4′-H), 4.01 (s , $CH_3O$), 4.35 (m, 3′-H), 4.90 (t, 5′-OH, J = 5 Hz), 5.29 (d, 3′-OH, J = 4 Hz), 6.48 (d, 5-H, J = 4 Hz), 6.55 (t, 1′-H, J = 5 Hz), 7.47 (d,6-H, J = 4 Hz).

| | | | | | |
|---|---|---|---|---|---|
| $C_{13}H_{17}N_3O_4S$ (311.4) | Ber.<br>Gef. | C 50.15<br>C 50.28 | H 5.50<br>H 5.47 | N 13.50<br>N 13.56 | S 10.30<br>S 10.31 |

b) 2-Mthylthio-6-methoxy-7-desaza-2′,3′-didesoxy-9-β-D-ribofuranosyl-purin

Wird durch Deoxigenierung der nach a) erhaltenen 2′-Desoxy-Verbindungwie unter Beispiel 1c) beschrieben hergestellt.
UV (Methanol): $\lambda_{max}$ = 283, 236 nm (ϵ = 1300, 15500)

| | | | | |
|---|---|---|---|---|
| $C_{13}H_{17}N_3O_3S$ (295.4) | Ber.<br>Gef. | C 52.8<br>C 52.6 | H 5.75<br>H 5.70 | N 14.2<br>N 14.2 |

**Beispiel 9**

6-Methoxy-7-desaza-2′,3′-didesoxy-9-β-D-ribofuranosyl-purin

a) 6-Methoxy-7-desaza-2′-desoxy-9-β-D-ribofuranosyl-purin

Die Synthese der Verbindung erfolgt wie in Liebigs Ann. Chem. 1985, 1360 - 1366 beschrieben.

b) Das Didesoxy-Derivat kann durch Deoxigenierung der Verbindung aus 9a) wie in Beispiel 1c) beschrieben erhalten werden.

Ein alternativer Weg besteht in der Engtschwefelung von 2-Methylthio-6-methoxy-7-desaza-2′,3′-didesoxy-9-β-D-ribofuranosyl-purin aus Beispiel 8 ebenfalls nach Liebigs Ann. Chem. 1985, 1360 - 1366.
DC ($CH_2Cl_2$/MeOH 9:1): $R_f$ = 0.8
UV(MeOH): $\lambda_{max}$ = 261 nm (log(ϵ) = 3.86).
$^1$H-NMR (DMSO-$d_6$): δ = 2.04 (m, 3′-H); 2.24 (m, 2′-$H_b$); 2.40 (m, 2′-$H_a$); 3.55 (m, 5′-H); 4.04 (s, $OCH_3$); 4.07 (m, 4′-H); 4.93 (t, J = 5.5 Hz, 5′-OH); 6.47 (dd, J = 4.4 und 6.8 Hz, 1′-H); 6.55 (d, J = 3.7 Hz, 5-H); 7.66 (d, J = 3.7 Hz, 6-H); 8.42 (s, 2-H).

| | | | | |
|---|---|---|---|---|
| $C_{12}H_{15}N_3O_3$ (249.3) | Ber.<br>Gef. | C 57.8<br>C 57.8 | H 6.0<br>H 6.05 | N 16.8<br>N 16.65 |

Eine weitere Möglichkeit der Herstellung dieser Verbindung ist in Beispiel 24 i) beschrieben.

**Beispiel 10**

7-Desaza-2′,3′-didesoxy-9-β-D-ribofuranosyl-purin-6-on

Die Herstellung der Verbindung erfolgt über die 2′-Desoxy-Verbindungwie in Liebigs Ann. Chem. 1985, 312 - 320 beschrieben und anschließender Deoxigenierung wie unter Biepiel 1c).
UV (Methanol): $\lambda_{max}$ = 258, 280 nm (Schulter), (ϵ = 9200, 6400)
DC ($CH_2CL_2$/MeOH 9:1): $R_f$ = 0.5
$^1$H-NMR (DMSO-$d_6$): δ = 2.00 (m, 3′-H); 2.16 (m, 2′-$H_b$); 2.37 (m, 2′-$H_a$); 3.49 (dd, J = 4.9 und 11.6 Hz, 5′-H); 3.58 (dd, J = 4.2 und 11.6 Hz, 5′-H); 4.05 (m, 4′-H); 6.33 (dd, J = 4.2 und 6.9 Hz, 1′-H); 6.50 (d, J = 3.5 Hz, 5-H); 7.36 (d, J = 3.5 Hz, 6-H); 7.90 (s, 2-H).

| | | | | |
|---|---|---|---|---|
| $C_{11}H_{13}N_3O_3$ (235.2) | Ber.<br>Gef. | C 56.1<br>C 56.0 | H 5.5<br>H 5.3 | N 17.8<br>N 18.0 |

Eine weitere Möglichkeit der Herstellung dieser Verbindung ist in Beispiel 24 j) beschrieben.

**Beispiel 11**

7-Desaza-2′,3′-didesoxy-9-β-D-robofuranosyl purin-2,6-dion

Synthese erfolgt über die 2′-Desoxy-Verbindung wie in Liebigs Ann. Chem. 1985, 312 - 320 beschrieben und anschließender Deoxigenierung wie unter Beispiel 1c).
UV (Phosphatpuffer, pH = 7.0):$\lambda_{max}$ = 251, 280 nm ($\epsilon$ = 10500, 7400)

| | | | | |
|---|---|---|---|---|
| $C_{11}H_{13}N_3O_4$ (251.4) | Ber.<br>Gef. | C 52.5<br>C 52.3 | H 5.2<br>H 5.1 | N 16.7<br>N 16.5 |

**Beispiel 12**

2,6-Dimethoxy-7-desaza-2′,3′-didesoxy-9-β-D-riobfuranosyl-purin

Das Derivat wurde durch Phasentransferglycosylierung des entsprechenden Purins und anschließender Deoxigenierung wie unter Beispiel 1c) beschrieben, synthetisiert.
UV (Methanol): $\lambda_{max}$ = 257, 271 nm ($\epsilon$ = 7300, 7400)

| | | | | |
|---|---|---|---|---|
| $C_{13}H_{17}N_3O_4$ (279.3) | Ber.<br>Gef. | C 55.85<br>C 55.7 | H 6.1<br>H 6.1 | N 15.0<br>N 15.1 |

**Beispiel 13**

6-Amino-7-desaza-2′,3′-didesoxy-9-β-D-ribofuranosyl-purin-2-on

Die Verbindung wurde nach J. Chem. Soc., Perkin Trans. II 1986, 525 - 530, durch Phasentransfer-Glykosylierung von 2-Methoxy-6-amino-7-desaza-purin, anschließender Demethylierung und letztlicher Deoxigenierung analog Beispiel 1c) erhalten.
UV (Methanol): $\lambda_{max}$ 255, 305 nm ($\epsilon$ = 7600, 7200)

| | | | | |
|---|---|---|---|---|
| $C_{11}H_{14}N_4O_3$ (250.2) | Ber.<br>Gef. | C 52.7<br>C 52.75 | H 5.6<br>H 5.5 | N 22.4<br>N 22.3 |

**Beispiel 14**

2-Amino-7-desaza-7-methyl-2′,3′-didesoxy-9-β-D-ribofuranosyl-purin-6-on

Die Verbindung wurde über das in Liebigs Ann. Chem. 1984, 708 -721 beschriebene 2′-Desoxynucleosid mit nachfolgender Deoxigenierung wie in Beispiel 1c) beschrieben, synthetisiert.
UV (Methanol): $\lambda_{max}$ = 224, 264, 285 nm (Schulter) ($\epsilon$ = 22500, 10500, 6500)

| | | | | |
|---|---|---|---|---|
| $C_{12}H_{16}N_4O_3$ (264.3) | Ber.<br>Gef. | C 54.5<br>C 54.3 | H 6.05<br>H 6.1 | N 21.2<br>N 21.1 |

**Beispiel 15**

2-Amino-7-desaza-2′-3′-didesoxy-3′-azido-9-β-D-ribofuranosyl-purin-6-on

Die Verbindung wurde durch Glycosylierung von 2-Amino-7-desaza-purin-6-onmit dem nach Byatkina/Azhayev (Synthesis 1984, 961 -963) hergestellten Azido-Zucker hergestellt.
UV (Methanol): $\lambda_{max}$ = 261, 281 nm (Schulter) ($\epsilon$ = 13300, 7800)

| | | | | |
|---|---|---|---|---|
| $C_{11}H_{13}N_7O_3$ (291.3) | Ber.<br>Gef. | C 45.3<br>C 45.4 | H 4.45<br>H 4.3 | N 33.65<br>N 33.4 |

**Beispiel 16**

3,7-Didesaza-2',3'-didesoxy-3'-azido-9-β-D-ribofuranosyl-purin

Die Verbindung wurde durch Ribosidierung von 3,7-Didesaza-purin mit dem nach Byatkina/Azhayev (Synthesis 1984, 961 - 963) herstellten Azido-Zucker präpariert.
UV (Methanol): $\lambda_{max}$ 224, 274 nm

| | | | | |
|---|---|---|---|---|
| $C_{12}H_{13}N_5O_2$ (259.2) | Ber.<br>Gef. | C 55.55<br>C 55.4 | H 5.0<br>H 5.1 | N 27.0<br>N 26.8 |

**Beispiel 17**

6-Amino-8-aza-7-desaza-2',3'-didesoxy-9-β-D-ribofuranosyl-purin

(4-Amino-1-(2',3'-didesoxy-β-D-erythro-pentofuranosyl)-1H-pyrazolo[3,4-d]pyrimidin)

a) 4-Benzoylamino-1-(2'-desoxy-9-β-D-erythro-pentofuranosyl-5'-O-(4,4'-dimethoxytriphenylmethyl)-1H-pyrazolo[3,4-d]pyrimidin

6-Amino-8-aza-7-desaza-2'-desoxy-β-D-ribofuranosyl-purin wurde hergestellt, wie in Helv. Chim. Acta 68, 563 - 570 (1985) beschrieben. Die Benzoylierung der 4-Aminogruppe und die anschließende Einführung der Dimethoxytritylschutzgruppe wurde analog zu bekannten Methoden durchgeführt.

b) 4-Benzoylamino-1-(2'-desoxy-β-D-erythro-pentofuranosyl)-5'-O-(4,4'-dimethoxytriphenylmethyl)-3'-O-phenoxythiocarbonyl-1H-pyrazolo[3,4-d]pyrimidin

200 mg (0,3 mmol) des Produkts aus Beispiel 17 a) wurden in 4 ml Acetonitril mit 82 µl (0.6 mmol) Phenylchlorothiocarbonat bei Raumtemperatur 16 Stunden lang in Anwesenheit von 90 mg (0,75 mmol) 4-(Dimethylamino)-pyridin umgesetzt. Nach chromatographischer Reinigung (Kieselgel, Dichlormethan/Ethylacetat 95:5) wurden 150 mg (63 %) des Produkts isoliert.
DC (Kieselgel, $CH_2Cl_2$/Ethylacetat, 95:5): $R_f$ = 0.4.
$^1$H-NMR ([$D_6$]DMSO) : $\delta$ = 3.26 (m, 5′-H), 3.69 (s, 2 x $OCH_3$), 4.45 (m, 4′-H), 5.98 (m, 3′-H), 8.45 (s, 3-H), 8.78 (s, 6-H), 11.72 (s, NH).

c) 4-Benzoylamino-1-(2′,3′-dideswoxy-9-β-D-glycero-pentofuranosyl)-5′-O-(4,4′-dimethoxytriphenylmethyl)-1H-pyrazolo-[3,4-d]pyrimidine

200 mg (0.25 mmol) des Produkts aus Beispiel 17 b) wurden nach Barton in 7 ml Toluol mit 150 µl (0,55 mmol) Tri-N-butylstannan und 15 mg 2,2′-azobis(2-methylpropionitril) während einer Stunde bei 80 °C unter Argon desoxigeniert. Nach Chromatographie (Kieselgel, Dichlormethan/Ethylacetat 95:5) erhielt man 120 mg (75 %) des Produkts (farblos, amorph).

DC (Kieselgel, $CH_2Cl_2$/Ethylacetat, 95:5) : $R_f$ = 3.0.
$^1$H-NMR ([$D_6$[DMSO):$\delta$ = 2.16 (m, 3′-H), 2.49 (m, 2′-H), 2.99 (m, 5′-H), 3.65, 3.68 (2s, 2 x $OCH_3$), 4.32 (m, 4′-H), 6.69 (m, 1′-H), 8.41 (s, 3-H), 8.80 (s, 6-H), 11.66 (s, NH).

d) 6-Amino-8-aza-7-desaza -2′,3′-didesoxy -9-$\beta$-D-ribofuranosyl-purin

(4-Amino-1-(2′,3′-didesoxy-$\beta$-D-glycero-pentofuranosyl)-1H-pyrazolo[3,4-d]pyrimidin)

a) 300 mg (0.47 mmol) des Produkts aus Beispiel 17 c) wurden in 40 ml mit Ammoniak gesättigtem Methanol bei 60 °C während 4 Stunden behandelt und zur Trockne eingedampft. Man erhält 200 mg (81 %) 4-Amino-1-(2′,3′-didesoxy-$\beta$-D-glyceropentofuranosyl)-5′-O-(4,4′-dimethoxytriphenylmethyl)-1H-pyrazolo[3,4-d]pyrimidin als farblosen Schaum nach Chromatographie an Kieselgel (Dichlormethan/Aceton 7:3).

DC(Dieselgel, $CH_2Cl_2$/Aceton, 8:2): $R_f$ = 0.25. $^1$-H-NMR ([$D_6$]DMSO): $\delta$ = 2.16 (m, 3′-H), 2.45 (m, 2′-H), 2.99 (m, 5′-H), 3.69, 3.70 (2s, 2 x $OCH_3$), 4.25 (m, 4′-H), 6.52 (m, 1′-H), 7.74 (s, $NH_2$), 8.06 (s, 3-H), 8.24 (s, 6-H).

b) 110 mg (0.2 mmol)n des Produkts wurden mit 10 ml 80 %iger Essigsäure bei Raumteperatur 20 Minuten lang gerührt. Nach Chromatographie (Kieselgel, Dichlormethan/Methanol 9:1) erhielt man das gewünschte Produkt kristallin. Nachfolgende Umkristallisation aus Isopropanol/Cyclohexan ergab 40 mg (85 %) des Produkts als farblosen Feststoff.

UV (MeOH): $\lambda_{max}$ = 260, 275 nm ($\epsilon$ = 9000, 10200)

| | | | | |
|---|---|---|---|---|
| $C_{10}H_{13}N_5O_2$ (235,25): | Ber: | C 51.06 | H 5,57 | N 29,77 |
| | Gef: | C 50,96 | H 5,65 | N 29,80 |

$^{13}$C-NMR ([$D_6$]DMSO): $\delta$ = 133 (C-8), 100.3 (C-5), 158.1 (C-6), 156.1 (C-2), 153.6 (C-4), 84.4 (C-1′), 30.4 (C-2′), 27.4 (C-3′), 81.7 (C-4′), 64.3 (C-5′) DC (Kieselgel, $CH_2Cl_2$/Methanol, 9:1): $R_f$ = 0.4. - UV (MeOH): $\lambda_{max}$ = 260, 275 nm ($\epsilon$ = 9000, 10200). $^1$H-NMR ([$D_6$)DMSO): $\delta$ = 2.11 (m, 3′-H), 2.40 (m, 2′-H), 3.36 (m, 1′-H), 4.08 (m, 4′-H), 4.75 (m, 5′-OH), 6.45 (m, 1′-H), 7.75 (s, $NH_2$), 8.14 (s, 3-H), 8.18 (s, 6-H).

## Beispiel 18

a) 4,6-Dichlor-1-(2′-desoxy-3′,5′-di-O-p-toluoyl-$\beta$-D-erythropento-furanosyl)-1H-pyrrolo[3,2-c]pyridin

Eine Lösung von 300 mg (1.6 mmol) 4,6-Dichloro-1H-pyrrolo[3,2-c]pyfidin in trockenem Acetonitril (75 ml), die 450 mg (8.0 mmol) Kaliumhydroxid und 30 mg (0.1 mmol) Tris-[2-(2-methoxyethoxy)ethyl]amin enthält, wurde bei Raumtemperatur unter Stickstoff 30 Minuten lang gerührt. Unter Rühren wurden 625 mg (1.6 mmol) $\alpha$-Chlor-2-desoxy-3,5-di-O-p-toluoyl-D-erythropentofuranose zugegeben und weitere 15 Minuten gerührt. Unlösliches Material wurde abfiltriert. Das Filtrat wurde im Vakuum eingeengt. Der ölige Rückstand wurde an Kieselgel (Säule 17 x 4 cm, Elutionsmittel Dichlormethan-Ethylacetat (97:3)) chromatographiert. Man erhielt 762 mg (90 %) des farblosen amorphen Produktes.

$^1$H-NMR ($Me_2SO-d_6$):$\delta$ = 2.37 und 2.41 (2s, 2 $CH_3$), 2.77 (m, H-2′s), 2.94 (m, H-2′ ), 4.57 (m, H-4′,H-5′), 5.68 (m, H-3′), 6.66 (pt. H-1′), 6.71 (d, J = 3.5 Hz, H-3), 8.00 (s, H-7),
$^{13}$C-NMR ($Me_2SO-d_6$):$\delta$ = 36.8 (C-2′), 64.2 (C-5′), 74.9 (C-3′), 81.7 (C-1′), 85.6 (C-4′), 102.0 (C-3), 106.1 (C-7), 123.1 (C-3a), 129.7 (C-2), 140.0 (C-6), 140.6 (C-4), 142.4 (C-7a).

b) 4,6-Dichlor-1-(2′-desoxy-$\beta$-D-erythro-pentofuranosyl)-1H-pyrrolo[3.2-c]pyridin

500 mg (0.93 mmol) der Verbindung aus Beispiel 18 a) wurden in 30 ml methanolischem Ammoniak gelöst und bei 50 °C 12 Stunden lang gerührt. Die Lösung wurde zur Trockne eingeengt, der feste Rückstand an Kieselgel 60H (2 g) adsorbiert und auf eine Kieselgelsäule (14 × 4 cm, Elutionsmittel Chloroform/Methanol (9:1)) aufgegeben. Aus der Hauptfraktion wurde des Produkt als ein farbloses Öl isoliert, welches aus wässrigem Ethanol in farblosen Nadeln kristallisierte.

Ausbeute: 101 mg (72 %), Schmp 180 °C
$^1$H-NMR ($Me_2SO-d_6$):$\delta$ = 2.28 (m, H-2′s), 2.43 (m, H-2′a), 3.56 (m, H = 5′), 3.85 (m, H-4′), 4.38 (m, H-3′), 5.02 (t, J = 5.2 Hz, 5′-OH), 5.34 (d, J = 4.1 Hz, 3′-OH), 6.42 (pt, H-1′), 6.67 (d, J = 3.4 Hz, H-3), 7.89 (d, J = 3.4 Hz, H-2), 7.96 (s, H-7). $^{13}$C-NMR ($Me_2SO-d_6$):$\delta$ = 40.6 (C-2′), 61.5 (C-5′), 70.5

(C-3′), 85.5 (C-1′), 87.6 (C-4′), 101.3 (C-3), 106.1 (C-7), 123.1 (C-3a), 129.7 (C-2), 139.7 (C-6), 140.4 (C-4), 142.0 (C-7a).

c) 4-Amino-6-chlor-1-(2′-desoxy-β-D-erythro-pentofuranosyl)-1H-pyrrolo[3,2-c]pyridin

460 mg (1.52 mmol) der Verbindung aus Beispiel 18 b) wurden in 6 ml trockenem Hydrazin gelöst und auf 80 °C 60 Mintuen lang erhitzt. Das Hydrazin wurde im vakuum entfernt und der ölige Rückstand zweimal mit je 10 ml Ethanol eingeengt. Der Rückstand wurde in 40 ml wässrigem Ethanol gelöst. Dann wurden 2 g Raney-Nickel-Katalysator zugegeben und die Mischung für zwei Stunden unter Rühren zum Sieden erhitzt. Der Katalysator wurde abfiltriert und gründlich mit heißem wässrigem Ethanol gewaschen.

Das Filtrat wurde zur Trockne eingeengt, der Rückstand in Methhanol gelöst, an 2 g Kieselgel adsorbiert und das Lösungsmittel im Vakuum entfernt. Dieses Kieselgel wurde in Chloroform/Methanol (9:1) suspendiert und auf eine Kieselgelsäule (6 x 3 cm) aufgegeben. Elution mit Chloroform/Methanol (9:1) ergab einen farblosen Sirup, aus welchem durch Kristallisation aus Methanol das Produkt in kleinen, farblosen Kristallen mit dem Schmelzpunkt 232 °C erhalten werden konnte.

Ausbeute: 207 mg, 48 %

DC (Chloroform/Methanol (9:1)): $R_f$ = 0.2; UV (Methanol) $\lambda_{max}$ = 277 nm (ε = 14800), 285 nm (ε = 13800); $^1$H-NMR ($Me_2SO-d_6$): δ = 2.20 (m, H-2′m), 2.40 (m, H-2′a), 3.51 (m, H-5′), 3.78 (m, H-4′), 4.32 (m, H-3′), 4.89 (t, J = 5 Hz, 5′-OH), 5.26 (d, J = 4 Hz, 3′-OH), 6.19 (pt. H-1′), 6.55 (s, $NH_2$), 6.64 (d, J = 3 Hz, H-3), 6.83 (s, H-7), 7.36 (d, J = 3 Hz, H-2).

$^{13}$C-NMR ($Me_2SO-d_6$): δ = 40 (C-2′), 61.8 (C-5′), 70.6 (C-3′), 84.7 (C-1′), 87.2 (C-4′), 95.1 (C-7), 101.6 (C-3), 109.6 (C-3a), 123.5 (C-2), 141.0 (C-6), 141.4 (C-7a, 152.9 (C-4).

| | | | | | |
|---|---|---|---|---|---|
| $C_{12}H_{14}ClN_3O_3$: | ber. | C, 50.80; | H, 4.97; | N, 14.81; | Cl. 12.50 % |
| | gef. | C, 50.91; | H, 5.05; | N. 14.75; | Cl. 12.53 % |

d) 4-Amino-1-(2′-desoxy-β-D-erythro-pentofuranosyl)-1H-pyrrolo[3,2-c]pyridin

Eine Lösung von 200 mg (0.7 mmol) der Verbindng aus Beispiel 18 c) in Methanol (30 ml), das 0.4 ml mit Ammoniak gesättigtem Methanol enthält, wurde in Gegenwart von Palladium-Tierkohle (50 mg, 10 % Pd) bei Raumtemperatur 30 Stunden lang hydriert. Der Katalysator wurde abfiltriert und das Lösungsmittel im Vakuum entfernt. Reinigung durch Flashchromatographie (Säule 4 x 4 cm, Elutionsmittel Chloroform/Methaol/Triethylamin (7:3:2)) und Kirstallisation aus Methaol ergaben 70 mg (40 %) des Produktes als farblose Kristalle vom Schmelzpunkt 205 °C.

DC (Laufmittel Chloroform/Methanol/Triethylamin (7:3:2) v/v/v): $R_f$ = 0.4; UV (Methanol) $\lambda_{max}$ = 271 nm (ε = 12800); $^1$H-NMR ($Me_2SO-d_6$): δ = 2.20 (m, H-2′-b), 2.42 (m, H-2′a), 3.51 (m, H-5′), 3.80 (m, H-4′), 4.32 (m, H-3′), 4.91 (m, 5′-OH), 5.32 (m, 3′-OH), 6.08 (s, $NH_2$), 6.23 (pt. H-1′), 6.65 (d, J = 3 Hz, H-3), 6.75 (d, J = 6 Hz, H-7), 7.35 (d, J = 3 Hz, H-2), 7.55 (d, J = 6 Hz, H-6).

$^{13}$C-NMR ($Me_2SO-d_6$): δ = 39.8 (C-2′), 62.0 (C-5′), 70.8 (C-3′), 84.5 (C-1′), 87.1 (C-4′), 96.9 (C-7), 101.5 (C-3), 110.7 (C-3a), 122.5 (C-2), 139.7 (C-6), 140.0 (C-7a), 153.7 (C-4).

| | | | | |
|---|---|---|---|---|
| $C_{12}H_{15}N_3O_3$: | ber. | C, 57.82; | H, 6.07; | N, 16.86 % |
| | gef. | C, 57.97; | H, 6.12; | N, 16.74 % |

**Beispiel 19**

a) 6-Chlor-1-(2′-desoxy-β-D-erythro-pentofuranosyl)-1H-pyrrolo[3.2-c]pyridin-4-on

Eine Lösung von 400 mg (1.32 mmol) der Verbindung aus Beispiel 18 b) in 2 N wässriger Natronlauge (mit geringen Mengen von 1,4-Dioxan) wurde 30 Stunden zum Sieden erhitzt. Die Reaktionsmischung wurde mit 2 N Salzsäure neutralisiert, filtriert und dann auf eine Amberlite-XAD-4-Säule (17 x 2 cm) aufgegeben. Anorganische Salze wurden durch Waschen mit Wasser entfernt. Das Produkt wurde mit Methanol eluiert. Kristallisation aus Wasser ergab 158 mg (42 %) farblose Kristalle. Schmelzpunkt 242 - 243

°C. DC (Chloroform/Methanol (8:2):
$R_f$ = 0.5 -UV (Methanol):$\lambda_{max}$ = 270 nm ($\epsilon$ = 11100), 292 nm ($\epsilon$ = 9300);
$^1$H-NMR ($Me_2SO-d_6$):$\delta$ = 2.22 (m, H-2′b), 2.38 (m, H-2′a), 3.53 (m, H-5′), 3.80 (m, H-4′), 4.33 (m, H-3′), 4.96 (m, 5′-OH), 5.29 (m, 3′-OH), 6.22 (pt. H-1′), 6.54 (d, J = 3.3 Hz, H-3), 6.96 (s, H-7), 7.38 (d, J = 3.3 Hz, H-2), 11.81 (br. NH). $^{13}$C-NMR ($Me_2SO-d_6$): 40.5 (C-2′), 61.7 (C-5′), 70.6 (C-3′), 85.0 (C-1′), 87.4 (C-4′), 94.9 (C-7), 104.1 (C-3), 114.0 (C-3a), 123.2 (C-2), 129.1 (C-6), 139.2 (C-7a), 158.7 (C-4).

| | | | | | |
|---|---|---|---|---|---|
| $C_{12}H_{13}ClN_2O_4$: | ber.<br>gef. | C, 50.63;<br>C, 50.79; | H, 4.60;<br>H, 4.74; | N, 9.84;<br>N, 9.80; | Cl, 12.45<br>Cl, 12.69 |

b) 1-(2′-Desoxy-$\beta$-D-erthyro-pentofuranosyl)-1H-pyrrolo [3,2-c]pyridin-4-on

Eine Lösung von 100 mg (0.35 mmol) der Verbindung aus Beispiel 19 a) in Methanol (15 ml) wurde mit 0.5 ml 25 %igem wässrigem Ammoniak vermischt und in Gegenwart von palladium/Tierkohle (10 % Pd, 15 mg) 3 Stunden lang bei Raumtemperatur hydriert. Der Katalysator wurde abfiltriert und das Filtrat zur Trockne eingeengt. Der feste Rückstand wurde aus Wasser kristallisiert.

Man erhielt 51 mg (58 %) Produkt vom Schmelzpunkt 147 - 148 °C. DC (Laufmittel Chloroform/Methanol (8:2):
$R_f$ = 0.3; -UV (Methanol):$\lambda_{max}$ = 264 nm ($\epsilon$ = 11700), 282 nm (sh, $\epsilon$ = 8000), 295 nm (sh, $\epsilon$ = 1500); -$^1$H-NMR ($Me_2SO-d_6$): $\delta$ = 2.22 (m, H-2′s), 2.40 (m, H-2′s), 3.52 (m, H-5′), 3.81 (m, H-4′), 4.32 (m, H-3′), 4.93 (t, J = 5.4 Hz, 5′-OH), 5.32 (d, J = 4.3 Hz, 3′-OH), 6.21 (pt. H-1′), 6.54 (d, J = 3 Hz, H-3), 6.62 (d, J = 7 Hz, H-7), 7.03 (d, J = 7 Hz, H-6), 7.34 (d, J = 3 Hz, H-2), 10.87 (br. NH).
$^{13}$C-NMR ($Me_2SO-d_6$):$\delta$ = 40 (C-2′, überlagert von Lösungsmittel-Signalen), 61.8 (C-5′), 70.7 (C-3′), 84.8 (C-1′), 87.4 (C-4′), 93.8 (C-7), 104.6 (C-3), 115.9 (C-3a), 122.0 (C-2), 127.8 (C-6), 139.0 (C-7a), 159.6 (C-4).

| | | | | |
|---|---|---|---|---|
| $C_{13}H_{14}N_2O_4$: | ber.<br>gef. | C, 59.08;<br>C, 59.09; | H, 6.10;<br>H, 6.07; | N, 10.60 %<br>N, 10.65 % |

**Beispiel 20:**

a) 1-(2′-Desoxy -$\beta$-D-erythro-pentofuranosyl)-4,6dichlor-5′-O-(4,4′-dimethoxytrityl)-1H-pyrrolo[3,2-c]pyridin

500 mg (1.65 mmol) der Verbindung aus Beispiel 18 b) wurden mit 10 ml Pyridin zur Trockne eingeengt. Das Material wurde in 10 ml trockenem Pyridin gelöst; man gab 0.7 ml (4.1 mmol) Hünig's Base sowie 690 mg (2.0 mmol) 4,4′-Dimethoxytritylchlorid zu. Die Lösung wurde für eine Stunde bei Raumtemperatur gerührt. Nach Zugabe von 75 ml 5 %iger wässriger Natriumbicarbonat-Lösung wurde mit Dichlormethan extrahiert (2 x 75 ml). Die vereinigten organischen Phasen wurden über Natriumsulfat getrocknet. Das Natriumsulfat wurde abfiltriert und das Filtrat eingeengt. Der Rückstand wurde auf eine Kieselgelsäule (30 x 3 cm, Elutionsmittel Dichlormethan und Dichlormethan/Aceton (99:1)) aufgegeben. Das Produkt wurde aus der Hauptfraktion als gelbliche amorphe Masse gewonnen. Das Produkt wurde in Ether gelöst und mit n-Hexan gefällt.
Ausbeute: 740 mg (74 %).

$^1$H-NMR ($Me_2SO-d_6$):$\delta$ = 2.39 (m, H-2′b), 2.64 (m, H-2′a), 3.09 (m, H-5′), 3.72 (s, 2 $OCH_3$), 3.96 (m, H-4′), 4.42 (m, H-3′), 5.41 (d, J = 4.8 Hz, 3′-OH), 6.47 (pt, H-1′), 6.65 (d, J = 3.5 Hz, H-3), 6.76 - 7.27 (aromat. H), 7.76 (d, J = 3.5 Hz, H-2), 7.89 (s, H-7),
$^{13}$C-NMR ($Me_2SO-d_6$):$\delta$ = 40 (C-2′ überlagert von Lösungsmittel Signalen), 55.1 (2 $OCH_3$), 63.6 (C-5′), 70.05 (C-3′), 85.0, 85.5, 85.5 (C-1′, C-4′, OCDMT), 101.3 (C-3), 106.2 (C-7), 123.2 (C-3a), 129.1 (C-2), 139.8 (C-6), 140.5 (C-4), 142.3 (C-7a).

| | | | | | |
|---|---|---|---|---|---|
| $C_{33}H_{30}Cl_2N_2O_5$: | ber.<br>gef. | C, 65.46;<br>C, 65.47; | H, 4.99;<br>H, 5.09; | Cl, 11.71;<br>Cl, 11.78; | N, 4.63 %<br>N, 4.56 % |

b) 1-(2′-Desoxy-β-D-erythro-pentofuranosyl)-4,6-dichlor-5′-O-(4,4′-dimethoxytrityl)-3′-O-phenoxythiocarbonyl-1H-pyrrolo [3,2-c]pyridin

300 mg (0.5 mmol) der Verbindung aus Beispiel 20 a) wurden in trockenem Acetonitril (11 ml) gelöst. 350 mg (2.8 mmol) 4-Dimethylaminopyridin und 150 µl (1.1 mmol) Phenylchlorthiocarbonat wurden zugegeben und die Lösung wurde bei Raumtemperatur 16 Stunden lang gerührt. Anschließend wurde die Reaktionsmischung im Vakuum zur Trockne eingeengt. Der Rückstand wurde an Kieselgel chromatographiert (Elutionsmittel Dichlormethan). Aus der Hauptfraktion wurde das farblose Produkt isoliert (310 mg. 84 %).

$^1$H-NMR ($Me_2SO$-$d_6$): δ = 2.92 (m, H-2′a,b), 3.35 (m, H-5′), 3.72 (s, 2 $OCH_3$), 4.43 (m, H-4′), 5.89 (m, H-3′), 6.61 (pt. H-1′), 6.71 (d, J = 3.5 Hz, H-3), 6.81-7.52 (aromat. H), 7.76 (d, J = 3.5 Hz, H-2), 8.01 (s, H-7).

$^{13}$C-NMR ($Me_2SO$-$d_6$): δ = 37.0 (C-2′), 55.1 (2 $OCH_3$), 63.8 (C-5′), 83.0, 84.2, 85.6. 86.0 (C-1′, C-3′, C-4′, O<u>C</u>DMT), 101.8 (C-3), 106.3 (C-7), 123.1 (C-3a), 128.9 (C-2), 140.1 (C-6), 140.6 (C-4), 142.4 (C-7a), 193.8 (<u>C</u>=S).

| | | | | | | |
|---|---|---|---|---|---|---|
| $C_{40}H_{34}Cl_2N_2O_6S$: | ber. | C 64,78; | H 4,62; | Cl 9,55; | N 3,77; | S 4,32 % |
| | gef. | C 64,66; | H 4.59; | Cl 9,65; | N 3.70; | S 4,40 % |

c) 4,6-Dichlor-1-(2′,3′-didesoxy-β-D-glycero-pentofuranosyl)-5′-O-(4,4′-dimethoxytrityl)-1H-pyrrolo[3,2-c]-pyridin

170 mg (0.23 mmol) der Verbindung aus Beispiel 20 b) und 15 mg (0.1 mmol)( 2,2′-azobis(2-methyl)-propionitril wurde in 10 ml trockenem Toluol unter Argonatmosphäre gelöst. Unter Rühren wurden 140 µl (0.51 mmol) Tri-n-butylstannan zugegeben und 3 Stunden lang bei 80 °C gerührt. Das Lösungsmittel wurde im Vakuum entfernt und der Rückstand an Kieselgel (Elutionsmittel Dichlormethan) chromatographiert. Aus der Hauptfraktion wurden 115 mg (85 %) des Produkts isoliert.

$^1$H-NMR ($Me_2SO$-$d_6$): δ= 2.05 (H-3′), 2.50 (H-2′, überlagert von Signalen des Lösungsmittels), 2.90 - 3.15 (m, H-5′), 4.25 (m, H-4′), 6.38 (m, H-1′), 6.63 (d, J = 3.4 Hz, H-3), 6.69 - 7.30 (aromat. H). 7.79 (d, J = 3.4 Hz, H-2), 7.89 (s, H-7).

d) 2,6-Dichlor-3,7-didesaza-2′,3′-didesoxy-9-β-D-ribofuranosyl-purin

Die Dimethoxytritylschutzgruppe der Verbindung aus Beispiel 20 c) wurde analog zu Beispiel 24 f) entfernt.

e) 6-Amino-3,7-didesaza-2′,3′-didesoxy-9-β-D-ribofuranosyl-purin

Die Verbindung aus Beispiel 20 d) wurde mit Hydrazin behandelt und anschließend mit Raney-Nickel reduziert wie in Beispiel 18 c) beschrieben. Man erhielt so die in Beispiel 1 D) beschriebene Verbindung.

f) 3,7-Didesaza-2′,3′-didesoxy-9-β-D-ribofuranosyl-purin

Die Verbindung aus Beipsiel 20 d) wurde mit Pd/Tierkohle/Wasserstoff analog zu Beispiel 24 g) hydriert. Man erhielt die schon in Beispiel 1 A) beschirebene Verbindung.

g) 3,7-Didesaza-2′,3′-didesoxy-9-β-D-ribofuranosyl-purin-6-on

Die Verbindung aus Beispiel 20 d) wurde mit Natronlauge behandelt wie in Beispiel 19 a) beschrieben und anschließend hydriert wie unter Beispiel 19 b) beschrieben. Man erhielt so die schon in Beispiel 1 E) beschirebene Verbindung.

**Beispiel 21:**

2-Amino-(2′,3′-didesoxy-β-D-glycero-pentofuranosyl)-1H-pyrazolo[3,4-d]pyrimidin-4-on

Deise Verbindung wurde analog zu dem in Beispiel 17 beschriebenen Weg über 2-Amino-(2′-desoxy-9-β-D-ribofuranosyl)-1H-pyrazolo[3,4-d]pyrimidin-4-on und Barton-Deoxygenierung von 2-Amino-(2′-desoxy-3′-O-methoxythiocarbonyl-5′-toluoyl-ribofuranosyl)-1H-pyrazolo[3,4-d]pyrimidin-4-on hergestellt.

| | | | | |
|---|---|---|---|---|
| $C_{10}H_{13}N_5O_3$ (251.25) | ber.:<br>gef.: | C 47.81<br>C 48.01 | H 5.22<br>H 5.30 | N 27.88 %<br>N 27.83 % |

$^{13}$C-NMR (DMSO-$d_6$):δ = 135.1 (C-3), 99.7 (C-3a), 157.9 (C-4), 155.3 (C-6), 154.5 (C-7a), 83.8 (C-1′), 30.3 (C-2′), 27.3 (C-3′), 81.6 (C-4′), 64.3′ (C-5′).
$^1$H-NMR: δ = 6.19 (dd, 1′-H, j = 6.9, 3.5 Hz), 2.06 (m, 3′-H)
Schmp.: 221 °C.

**Beispiel 22:**

3,7-Didesaza-2′-desoxy-9-β-D-ribofuranosyl-purin (2′-Desoxy-3,7-didesaza-nebularin)

Die Verbindung aus Beispiel 18 b) wurde an Palladium/Tierkohle (10 % Pd) in ammoniakalischem Methanol hydriert. Das Produkt wurde nach Abfiltrieren des Katalysators und Einengen des Filtrats im Vakuum von anorganischen Salzen durch Chromatographie an Amberlite XAD (Methanol/Wasser) sowie Kristallisation aus Wasser gereinigt. Schmp.: 175 - 176 °C
$^{13}$C-NMR ([$D_6$]DMSO: δ = 126.9 (C-2), 101.7 (C-3), 125.5 (C-3a), 143.3 (C-4), 140.6 (C-6), 105.9 (C-7), 139.2 (C-7a), 84.6 (C-1′), 70.8 (C-3′), 87.8 (C-4′), 61.9 (C-5′).
UV (0.1 N wässr. HCl): $\lambda_{max}$ = 224,274 nm

| | | | | |
|---|---|---|---|---|
| $C_{12}H_{14}N_2O_3$ | ber.:<br>gef.: | C 61.53<br>C 61.55 | H 6.02<br>H 6.12 | N 11.96 %<br>N 12.02 % |

$^1$H-NMR (DMSO-$d_6$): δ = 2.23 (m, 2′-Hb), 2.29 (m, 2′-Ha), 3.55 (m, 5′-$H_2$, 3.85 (m, 4′-H0, 4.38 (m, 3′-H), 4.99 (5′-OH), 5.37 (3′-OH), 6.42 (pt, 1′-H), 6.66 (d, J = 3 Hz, 3-H), 7.62 (d, j = 6 Hz, 7-H), 7.71 (d, j = 3 Hz, 2-H), 8.21 (d, j = 6 Hz, 6-H), 8.23 (s, 4-H).

**Beispiel 23:**

a) 2-Chlor-6-methoxy-3,7-didesaza-2′-desoxy-9-β-D-ribofuranosyl-purin

Die Verbindung aus Beispiel 18 b) wurde 40 Stunden in 1 N methanolischer Natriummethanolatlösung erhitzt. Das Reaktionsprodukt wurde an Amberlite XAD durch hydrophobe Chromatographie gereinigt (Methanol/Wasser).
UV (Methanol): $\lambda_{max}$ = 271, 280 nm

| | | | | | |
|---|---|---|---|---|---|
| $C_{13}H_{15}ClN_2O_4$ | ber.<br>gef. | C 52.27<br>C 52.24 | H 5.06<br>H 5.14 | Cl 11.87<br>Cl 12.05 | N 9.38 %<br>N 9.46 % |

b) 2-Chlor-3,7-didesaza-2′-desoxy-9-β-D-ribofuranosyl-purin-6-on

30stündiges Erhitzen der Verbindung aus Beispiel 18 b) in 2N wässriger Natronlauge/1,4-Dioxan ergab 2-Chlor-3,7-didesaza-2′-desoxy-9-β-D-ribofuranosyl-purin-6-on:
UV (Methanol): $\lambda_{max}$ = 262 nm

| | | | | |
|---|---|---|---|---|
| $C_{13}H_{16}N_2O_4$: | ber.: | C 59.08 | H 6.10 | N 10.60 % |
| | gef.: | C 59.09 | H 6.07 | N 10.65 % |

$^1$H-NMR ([D$_6$]DMSO: $\delta$ = 2.22 (m, 2′-H$_b$) 2.38 (m, 2′-H$_a$), 3.53 (m, 5′-H$_2$), 3.80 (m, 4′-H), 4.33 (m, 3′-H), 4.96 (5′-OH), 5.29 (3′-OH), 6.22 (pt, 1′-H), 6.54 (d, j = 3 Hz, 3-H), 6.96 (s, 7-H), 7.38 (d, j = 3 Hz, 2-H), 11.81 (NH).

**Beispiel 24:**

a) 4-Chlor-7-(2′-desoxy-3,5-di-O-(p-toluoyl)-$\beta$-D-erythropentofuranosyl)-7H-pyrrolo[2,3-d]pyrimidin

1 g (17.8 mmol) pulverförmiges Kaliumhydroxid wurde bei Raumtemperatur in 60 ml trockenes Acetonitril gegeben. Unter Rühren wurden 100 µl (0.31 mmol) Tris-[2-(2-methoxy-ethoxy)ethyl]amin zugegeben. Nach 5 Minuten wurden 1.23 g (8.01 mmol) 4-Chlor-7H-pyrrolo[2,3-d]pyrimidin in der Reaktionsmischung gelöst und für weitere 5 Minuten gerührt. Dann wurde $\alpha$-Chlor-2-desoxy-3,5-di-O-p-toluoyl-$\beta$-D-erythro-pento-furanose zugegeben. Nach 15minütigem Rühren wurde unlösliches Material durch Filtration entfernt. Das Filtrat wurde im Vakuum zur Trockne eingeengt und der Rückstand an einer Kieselgelsäule (5 x 7 cm, Chloroform) chromatographiert. Einengen des Eluats im Vakuum ergab 3.26 g (81 %) Produkt, das aus Ethanol in farblosen Nadeln kristallisierte (Schmelzpunkt 120 °C).

Weitere Varianten des Herstellungsverfahrens:

(I) Fest-flüssig-glykosilierung in Abwesenheit eines Katalysators: Die Reaktion wurde ausgeführt wie oben beschrieben, jedoch ohne Katalysator. Nach Aufarbeiten erhielt man 2.82 g (70 %) des Produktes.
(II) Durch flüssig-flüssig-Phasentransfer-Glykosilierung: 500 mg (3.26 mmol) 4-Chlor-7H-pyrrolo[2,3-d]-pyrimidin wurden in 20 ml Dichlormethan gelöst. 9 ml 50 %iger wässriger Natronlauge wurde zugegeben. Nach Zugabe von 10 mg ((0.03 mmol) Tetrabutylammoniumhydrogensulfat wurde die Lösung mit einem Vibromischer eine Minute gerührt. Anschließend wurden 1.4 g (3.61 mmol) der oben beschriebenen Halgenose zugegeben und weitere drei Minuten gemischt. Danach wurden die Phasen getrennt. Die wässrige Phase wurde zweimal mit jeweils 25 ml Dichlormethan extrahiert. Die vereinigten organischen Phasen wurden über Natriumsulfat getrocknet. Das Filtrat wurde zur Trockne eingeengt. Der Rückstand wurde an Kieselgel (Säule 5 x 5 cm, Chloroform) chromatographiert. Isolierung des Materials der Hauptfraktion und Kristallisation aus Ethanol ergab 1.04 g (63 %) Produkt. Schmelzpunkt: 118 °C.
DC (Cyclohexan/Ethylacetat 3:2): $R_f$ = 0.7.

UV (MeOH): $\lambda_{max}$ = 240 nm (log($\epsilon$) = 4.48).
$^1$H-NMR (DMSO-d$_6$): $\delta$ = 2.37, 2.40 (s, 2 $CH_3$); 2.77 (m, 2′-H$_b$);3.18 (m, 2′-Ha); 4.60 (m,4′-H und 5′-H); 5.77 (m, 3′-H); 6.75 (d, J = 3.7 Hz, 5-H); 6.78 (m, 1′-H); 7.34, 7.91 (m, 8 aromat. H und 6-H); 8.65 (s, 2-H).

b) 4-Chlor-7-(2′-desoxy-$\beta$-D-erythro-pentofuranosyl)-7H-pyrrolo[2,3-d]pyrimidin

2.4 g (4.7 mmol) der Verbindung aus Beispiel 24 a) wurden in 100 ml mit Ammoniak gesättigtem Methanol für 24 Stunden bei Raumtemperatur gerührt. Die Lösung wurde zur Trockne eingeengt, der Rückstand an Kieselgel 60H (10 g) adsorbiert und auf eine Kieselgelsäule (4 x 10 cm, Chloroform/Methanol, 95:5) aufgegeben. Aus der Hauptfraktion wurde das Produkt als eine farblose feste Substanz isoliert, die aus Ethylacetat in farblosen Nadeln kristallisierte. Ausbeute: 1.07 g (84 %). Schmelzpunkt 162 °C. DC (Chloroform/Methanol, 9:1): $R_f$ = 0.6.
UV (MeOH): $\lambda_{max}$ = 273 nm (log $\epsilon$ = 3.69). $^1$H-NMR (DMSO-d$_6$): w = 2.28 (m, 2′-Hb); 2.58 (m, 2′-Ha); 3.57 (m, 5′-H); 3.87 (m, 4′-H); 4.40 (m, 3′-H); 5.00 (t, J = 5.4 Hz, 5′-OH); 5.35 (d, J = 4.2 Hz, 3′-OH); 6.66 (m, 1′-H); 6.72 (d, J = 3.8 Hz, 5-H); 7.99 (d, J = 32.8 Hz, 6-H); 8.66 (s, 2-H).

c) 4-Chlor-7-(2′-deoxy-$\beta$-D-erythro-pentofuranosyl-5′-O-(4,4′-dimethoxytrityl)-7H-pyrrolo[2,3-d]pyrimidin

1 g (3.7 mmol) der Verbindung aus Beispiel 24 b) wurden durch Einengen mit 10 ml trockenem Pyridin getrocknet. Das Material wurde in trockenem Pyridin (20 ml) gelöst. 2 ml (11.7 mmol) Hünig's Base und 2 g (5.9 mmol) 4,4′-Dimethoxytritylchlorid wurden zugegeben. Die Lösung wurde drei Stunden lang bei Raumtemperatur gerührt. Nach Zugabe von 80 ml 5 %iger wässriger Natriumbicarbonatlösung wurde die Lösung mit 3 x 100 ml Dichlormethan extrahiert. Die vereinigten organischen Phasen wurden über

Natriumsulfat getrocknet. Nach Abfiltrieren wurde das Filtrat im Vakuum eingeengt. Der Rückstand wurde säulenchromatyographisch gereinigt (Kieselgel, Elutionsittel Dichlormethan und Dichlormethan/Ethylacetat, 9:1). Isolierung des Materials der Hauptfraktion, Lösen in Ether und Fällen mit Petrolether ergab 1.66 g (78 %) gelbliche amorphe Substanz.

| | | | | | |
|---|---|---|---|---|---|
| $C_{32}H_{30}N_3O_5Cl$ (572.1) | ber.<br>gef. | C 67.19<br>C 67.03 | H 5.29<br>H 5.47 | Cl 6.20<br>Cl 6.19 | N 7.35 %<br>N 7.29 % |

DC ($CH_2Cl_2$/Aceton, 9:1): $R_f$ = 0.3.
UV (MeOH): $\lambda_{max}$ = 274 nm (log($\epsilon$) = 3.85).
$^1$H-NMR (DMSO-$d_6$): $\delta$ = 2.36 (m, 2′-$H_b$); 2.70 (m, 2′-$H_a$); 3.72 (s, $OCH_3$); 3.18 (d, J = 4.5 Hz, 5′-H); 3.99 (m, 4′-H); 4.45 (m, 3′-H); 5.42 (d, J = 4.6 Hz, 3′-OH): 6.65 (m, 1′-H); 6.69 (d, J = 3.7 Hz, 5-H); 7.81 (d, J = 3.7 Hz, 6-H), 8.64 (s, 2-H).

d) 4-Chlor-7(2′-desoxy-$\beta$-D-erythro-pentofuranosyl)-5′-O-(4,4′-dimethoxytrityl)-3′-O-phenoxythiocarbonyl-7H-pyrrolo[2,3-d]pyrimidin

1 g (1.7 mmol) der Verbindung aus Beispiel 24 c) wurden in 30 ml trockenem Acetonitril gelöst. 500 mg (4.1 mmol) 4-Diemthylaminopyridin und 400 µl (2.9 mmol) Phenylchlorothiocarbonat wurden zugegeben und die Lösung wurde bei Raumtemperatur 16 Stunden lang gerührt. Anschließend wurde die Reaktionsmischung im Vakuum zur Trockne eingeengt und der Rückstand auf eine Kieselgelsäule (3 × 15 cm, Dichlormethan) aufgegeben. Aus der Hauptfraktion wurden 950 mg (76 %) farbloses amorphes Produkt isoliert.

| | | | | | | |
|---|---|---|---|---|---|---|
| $C_{39}H_{34}ClN_3O_6S$ (708.2) | ber.<br>gef. | C 66.14<br>C 66.22 | H 4.84<br>H 4.94 | Cl 5.01<br>Cl 5.12 | N 5.93<br>N 5.93 | S 4.53<br>S 4.46 |

DC ($CH_2Cl_2$/Aceton 95:5) $R_f$ = 0.8.
UV (MeOH): $\lambda_{max}$ = 274 nm (log($\epsilon$) = 3.87).
$^1$H-NMR (DMSO-$d_6$): $\delta$ = 2.84 (m, 2′-$H_b$); 3.21 (m, 2′-$H_a$); 3.37 (m, 5′-H); 4.46 (m, 4′-H); 5.92 (m, 3′-H); 6.70 (m, 1′-H); 6.76 (d, J = 3.8 Hz, 5-H); 7.85 (d, J = 3.8 Hz, 6-H); 8.61 (s, 2-H).

e) 4-Chloro-7-(2′,3′-didesoxy-$\beta$-D-glycero-pentofuranosyl)5′-O-(4,4′-dimethoxytrityl)-7H-pyrrolo[2,3-d]-pyrimidin

800 mg (1.1 mmol) der Verbindung aus Beispiel 24 d) und 40 mg (0.2 mmol) 2,2′-Azobis(2-methyl)-propionitril wurden in 40 ml trockenem Toluol unter Argonatmosphäre gelöst. 600 µl (2.2 mmol) Tri-n-butylstannan wurden unter Rühren zugegeben und die Reaktion wurde fortgesetzt bei 75 °C für zwei Stunden. Das Lösungsmittel wurde im Vakuum entfernt und der Rückstand wurde an Kieselgel (Säule 15 × 3 cm, Dichlormethan/Ethylacetat 95:5) chromatographiert. Aus der Hauptfraktion erhielt man 470 mg (75 %) des Produktes.

| | | | | | |
|---|---|---|---|---|---|
| $C_{32}H_{30}ClN_3O_4$ (556.1) | ber.<br>gef. | C 69.12<br>C 69.07 | H 5.44<br>H 5.53 | Cl 6.38<br>Cl 6.33 | N 7.56 %<br>N 7.58 % |

DC ($CH_2Cl_2$/Aceton 95:5): $R_f$ = 0.5
UV (MeOH): $\lambda_{max}$ = 273 nm (log($\epsilon$) = 3.78).
$^1$H-NMR (DMSO-$d_6$): $\delta$ = 2.08 (m, 3′-H); 2.10 (m, 2′-$H_b$); 2.43 (m, 2′-$H_a$); 3.11 (d, J = 4.4 Hz, 5′-H); 3.71 (s, $OCH_3$); 4.27 (m, 4′-H); 6.55 (dd, J = 3.6 und 6.9 Hz, 1′-H); 6.64 (d, J = 3.7 Hz, 5-H); 7.83 (d, J = 3.7 Hz, 6-H); 8.67 (s, 2-H).

f) 4-Chlor-7-(2′,3′-didesoxy-$\beta$-D-glycero-pentofuranosyl-7H-pyrrolo[2,3-d]pyrimidin

400 mg (0.7 mmol) der Verbindung aus Beispiel 24 e) wurde in 15 ml 80 %iger wässriger Essigsäure gelöst und bei Raumtemperatur für 30 Minuten gerührt. Das Lösungsmittel wurde im Vakuum entfernt und

Spuren von Essigsäure durch Einengen mit Wasser entfernt. Der Rückstand wurde säulenchromatographisch (Dichlormethan und Dichlormethan/Methanol, 98:2) gereinigt. Aus der Hauptfraktion wurden 120 mg (67 %) Produkt nach Kristallisation aus Ethylacetat als farblose Nadeln erhalten. Schmelzpunkt 90 °C.

| | | | | | |
|---|---|---|---|---|---|
| $C_{11}H_{12}ClN_3O_2$ (253.7) | ber.<br>gef. | C 52.08<br>C 52.20 | H 4.77<br>H 4.81 | Cl 13.98<br>Cl 14.04 | N 16.56<br>N 16.54 |

DC ($CH_2Cl_2$/MeOH 95:54): $R_f$ = 0.5
UV (MeOH): $\lambda_{max}$ = 274 nm (log(ε) = 3.65).
$^1$H-NMR (DMSO-$d_6$): δ = 2.04 (m, 3′-H); 2.28 (m, 2′-$H_b$); 2.46 (m, 2′-$H_a$); 3.57 (m, 5′-H); 4.11 (m, 4′-H); 4.95 (t, J = 5.5 Hz, 5′-OH); 6.52 (dd, J = 3.8 und 6.9 Hz, 1′-H); 6.69 (d, J = 3.8 Hz, 5-H); 8.01 (d, J = 3.8 Hz, 6-H); 8.64 (s, 2-H).

g) <u>7-(2′,3′-Didesoxy-β-D-glycero-pentofuranosyl)-7H-pyrrolo [2,3-d]-pyrimidine</u>

Eine Lösung von 200 mg (0.8 mmol) der Verbindung aus Beispiel 24 f) wurden in 20 ml Methanol, welchem 0.5 ml (6.6 mmol) konzentriert wässriger Ammoniak zugesetzt worden waren, wurde mit Palladium auf Tierkohle (40 mg, 10 % Pd) in einer Wasserstoffatmosphäre bei Raumtemperatur für drei Stunden gerührt. Der Katalysator wurde abfiltriert und das Lösungsmittel im Vakuum entfernt. Der Rückstand wurde in Wasser gelöst und auf einer Amberlite XAD-4-Säule (1. Elutionsmittel Wasser, 2. Elutionsmittel Wasser/Methanol (8:2)) chromatographiert. Isolierung des Materials der Hauptzone ergab 130 mg (75 %) des farblosen Produkts in Nadeln. Schmelzpunkt 131 °C.

| | | | | |
|---|---|---|---|---|
| $C_{11}H_{13}O_2N_3$ (219.2) | ber.<br>gef. | C 60.26<br>C 60.19 | H 5.98<br>H 5.97 | N 19.17 %<br>N 19.18 % |

DC ($CH_2Cl_2$/MeOH 9:1): $R_f$ = 0.6
UV (MeOH): $\lambda_{max}$ = 270 nm (log(ε) = 3.56).
$^1$H-NMR (DMSO-$d_6$): δ = 2.06 (m, 3′-H); 2.27 (m, 2′-$H_b$); 2.42 (m, 2′-$H_a$); 3.55 (m, 5′-H); 4.09 (m, 4′-H); 4.93 (t, J = 5.5 Hz, 5′-OH); 6.54 (dd, J = 4.3 und 6.9 Hz, 1′-H); 6.67 (d, J = 3.7 Hz, 5-H); 7.86 (d, J = 3.7 Hz, 6-H); 8.79 (s, 4-H); 9.01 (s, 2-H).

h) <u>4-Amino-7-(2′,3′-didesoxy-β-D-glycero-pentofuranosyl)-7H-pyrrolo[2,3-d]pyrimidin(2′,3′-dideoxytubercidin)</u>

200 mg (0.8 mmol) der Verbindung aus Beispiel 24 f) wurden in 60 ml 25 %igem wässrigen Ammoniak 15 Stunden lang bei 100 °C unter Druck in einer Stahlbombe gerührt. Anschließend wurde das Lösungsmittel im Vakuum entfernt und der Rückstand in 200 ml Wasser gelöst. Diese Lösung wurde an Dowex 1 x 2 ($OH^-$ Form) gereinigt. Die Säule wurde mit Wasser gewaschen und das Produkt mit Wasser/Methanol (9:1) eluiert. Aus der Hauptzone wurden 120 mg (65 %) Produkt gewonnen.
DC ($CH_2Cl_2$/MeOH 9:1): $R_f$ = 0.3
$^1$H-NMR (DMSO-$d_6$): δ = 2.03 (m, 3′-H); 2.22 (m, 2′-$H_a$); 2.33 (m, 2′-$H_b$); 3.53 (m, 5′-H); 4.04 (m, 4′-H); 4.99 (m, 5′-OH); 6.35 (m, 1′-H); 6.51 (d, J = 3.6 Hz, 5-H); 7.00 (s, $NH_2$); 7.34 (d, J = 3.6 Hz, 6-H); 8.04 (s, 2-H).

i) <u>7-(2′,3′-didesoxy-β-D-glycero-pentofuranosyl)-4-methoxy-7H-pyrrolo[2,3-d]pyrimidin</u>

170 mg (0.7 mmol) der Verbindung aus Beispiel 24 f) wurden in 5 ml 1M methanolischer Methanolatlösung gelöst und bei Raumtemperatur vier Stunden gerührt. Die Lösung wurde mit 80 %iger Essigsäure neutralisiert, im Vakuum eingeengt und der Rückstand wurde auf eine Kieselgelsäule (Elutionsmittel Dichlormethan/Methanol, 98:2) aufgegeben. Isolieren der Hauptzone ergab ein farbloses Öl, welches bei Lagerung in Nadeln kristallisierte. Ausbeute: 130 mg (78 %)

j) <u>7-(2′,3′-Didesoxy-β-D-glycero-pentofuranosyl)-4H-pyrrolo[2,3-d]pyrimidin-4-on</u>

200 mg (0.8 mmol) der Verbindung aus Beispiel 24 f) wurden in 10 ml 2N Natronlauge suspendiert und fünf Stunden unter Rückfluß zum Sieden erhitzt. Die Lösung wurde mit 80 %iger Essigsäure neutralisiert

und das unlösliche Material durch Filtration entfernt. Das Filtrat wurde auf eine Amberlite XAD-4 Säule aufgegeben. Die Säule wurde mit 500 ml Wasser gewaschen und das Produkt mit Wasser/2-Propanol (9:1) eluiert. Man erhielt 180 mg (80 %) Produkt.

## Beispiel 25

1-(2′,3′-didesoxy-β-D-glycero-pentafuranosyl)-1H-pyrazolo[3,4-d]pyrimidin-4-on

Das Produkt aus Beispiel 17 d) wurde mit Adenosindeaminase aus intestinalen Kalb-Mucosa-Zellen deaminiert. Der Fortgang der Reaktion wurde bei 275 nm UV-spektroskopisch verfolgt. Die Reaktion leifert das Produkt quantitativ in Form farbloser Kristalle. Schmp. 171 °C
UV (MeOH): $\lambda_{max}$ = 251 nm ($\epsilon$ = 7700)
DC (Kieselgel, Dichlormethan/Methanol 9:1): $R_f$ = 0.5
$^{13}$C-NMR ([$D_6$]-DMSO): $\delta$ = 135,2 (C-8), 106.1 (C-5), 157.3 (C-6), 148.4 (C-2), 152.3 (C-4), 84.6 (C-1′), 30.7 (C-2′), 27.3 (C-3′), 82.2 (C-4′), 64.2 (C-5′).
$^{1}$H-NMR ([$D_6$]-DMSO): $\delta$ = 2.13 (m, 3′-H), 2.40 (m, 2′-H), 3.40 (m, 5′-H), 4.09 (m, 4′-H), 4.73 (m, 5′-OH), 6.43 (m, 1′-H), 8.11 (s,3-H), 8.13 (s,6-H).

## Beispiel 26

2-Amino-7-desaza-2′,3′-didesoxy-9-β-D-ribofuranosyl-purin-6-on-5′-triphosphat

| | | |
|---|---|---|
| $C_{11}H_{14}N_4O_{12}P_3Na_3$ (556.2) | Ber.<br>Gef. | P: 16.7<br>P: 16.4 |

UV (Puffer, pH 7.0): $\lambda_{max}$ 259 nm ($\epsilon$ - 13400)
$^{31}$P-NMR ($D_2O$): $\delta$ = - 8.35 (d, P-$\gamma$), - 10.0 (d, P-$\alpha$), - 21.5 (t, P-$\beta$)

## Beispiel 27

2-Amino-3,7-didesaza-2′-desoxy-9-β-D-ribofuranosyl-purin-6-on-5′-triphosphat

| | | |
|---|---|---|
| $C_{12}H_{15}N_3O_{13}P_3Na_3$ (555.2) | Ber.<br>Gef. | P: 16.75<br>P: 16.5 |

UV (Puffer, pH 7.0): $\lambda_{max}$ = 272 nm ($\epsilon$ = 12400)

## Beispiel 28

3,7-Didesaza-2′,3′-didesoxy-9-β-D-ribofuranosyl-purin-5′-triphosphat

| | | |
|---|---|---|
| $C_{12}H_{14}N_2O_{11}P_3Na_3$ (524.1) | Ber.<br>Gef. | P: 17.7<br>P: 17.3 |

UV (Puffer, pH 7.0): $\lambda_{max}$ = 224, 274 nm Sämtliche, in den Beispielen 26 bis 28 aufgeführten Triphosphate wurden durch Phosphorylierung der entsprechenden Nucleoside nach Yoshikawa (Tetrah. Lett. 50, 5065 (1967)) zum 5′-Monophosphat und anschließender Überführung in das 5′-Triphosphat nach Hoard und Ott (J. Am. Chem. Soc. 87, (1965) 1785) hergestellt.

**Beispiel 29**

Antivirale Aktivität

Die Stabilität der N-glycosidischen Bindung von 2′,3′-Didesoxynucleosiden ist verbunden mit der antiviralen Aktivität.

Die Hydrolyse der Bindung wurde untersucht bei 25 °C bei drei verschiedenen Konzentrationen an Salzsäure. Dazu wurde die Abnahme der UV-Absorption ($E_t$) bei 258 nm gemessen. Über die Absorptions/Zeit-Kurve wurden die Geschwindigkeitskonstanten des Hydrolyse (k) und die Halbwertszeiten (T/2) anhand der Gleichung

$$K = 1/t \cdot 1n\ (E_o - E_\infty)/(E_t - E_\infty)$$

ermittelt. Dabei ist $E_o$ die Absorption zur Zit t = o und $E_\infty$ die Absorption nach vollständiger Beendigung der Reaktion.

Verglichen wurden 2′,3′-Didesoxyadenosin (a) und 6-Amino-8-aza-7-desaza-2′,3′-didesoxy-9-β-D-ribofuranosyl-purin (b) bei 25 °C.

Tabelle 1

| | | 1 N HCl | 0.1 N HCl | 0.01 N HCl |
|---|---|---|---|---|
| (a) | T/2 | -- | 1.9 min | 31.5 min |
| | k | -- | 0.363 $min^{-1}$ | 0.022 $min^{-1}$ |
| (b) | T/2 | 0.83 min | 20.4 min | 280 min |
| | k | 0,85 $min^{-1}$ | 0.033 $min^{-1}$ | 0.0025 $min^{-1}$ |

Tabelle 1 zeigt, daß die erfindungsgemäße Verbindung (b) mehr als 10 mal stabiler und damit antiviral wirksamer ist als (a).

**Patentansprüche**

**1.** Desaza-purin-nucleosid-Derivate der Formel I

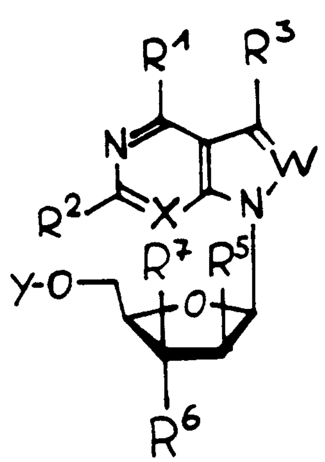

(I)

in der

| | |
|---|---|
| X | Stickstoff oder eine Methingruppe, |
| W | Stickstoff oder die Gruppe |

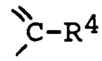

| | |
|---|---|
| $R^1$, $R^2$, $R^3$, $R^4$, | die gleich oder verschieden sein können, Wasserstoff, Halogen, eine Niederalkyl-, Hydroxy-, Mercapto-, Niederalkylthio-, Niederalkyloxy-, Aralkyl-, Aralkyloxy-, Aryloxy- oder eine, gegebenenfalls ein- oder zweifach substituierte, Aminogruppe, |
| $R^5$ | Wasserstoff oder eine Hydroxygruppe, |
| $R^6$, $R^7$ | jeweils Wasserstoff oder einer der beiden Reste $R^6$ und $R^7$ Halogen, eine Cyano-, eine Azido- oder eine, gegebenenfalls ein- oder zweifach substituierte, Aminogruppe bedeuten, wobei einer der Reste $R^6$ und $R^7$ auch eine Hydroxygruppe vorstellen kann, wenn X eine Methingruppe bedeutet, und außerdem $R^5$ und $R^7$ zusammen eine weitere Bindung zwischen C-2'und C-3' darstellen können und |
| Y | Wasserstoff, eine Monophosphat-, Diphosphat- oder Triphosphatgruppe vorstellt, mit Ausnahme der Verbindungen 2',3'-Didesoxy-tubercidin, 4,6-Dichloro-1-(2-desoxy-β-D-erythro-pentofuranosyl)pyrrolo[3,2-c]pyridin, 6-Amino-1-(2-desoxy-β-D-erythro-pentofuranosyl)pyrazolo[4,3-c]pyridin-4(5H)-on, 6-Amino-1-(2-desoxy-β-D-erythro-pentofuranosyl)-1H-pyrrolo[3,2-c]pyridin-4(5H)-on, 6-Amino-1-β-D-arabinofuranosyl-1H-pyrrolo[3,2-c]pyridin-4(5H)-on, 7-Iodo-2',3'-didesoxy-7-desazaguanosin, 2',3'-Didesoxy-2',3'-didehydro-7-desazaadenosin, 7-Iodo-2',3'-didesoxy-7-desazaadenosin, 7-Iodo-2',3'-didesoxy-7-desazainosin, |

sowie mögliche Tautomere und Salze und Nucleinsäuren, die eine oder mehrere Verbindungen der Formel I als Baustein enthalten.

**2.** Verbindungen nach Anspruch 1, dadurch gekennzeichnet, daß W die Gruppe

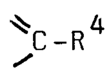

bedeutet.

**3.** Verbindungen nach Anspruch 1, dadurch gekennzeichnet, daß W Stickstoff bedeutet.

**4.** Verbindungen nach einem der Ansprüche 1-3, dadurch gekennzeichnet, daß X eine Methingruppe darstellt.

**5.** Verbindungen nach Anspruch 4, dadurch gekennzeichnet, daß einer der Reste $R^6$ und $R^7$ eine Hydroxygruppe bedeutet.

**6.** Verfahren zur Herstellung der Verbindungen nach den Ansprüchen 1-5, dadurch gekennzeichnet, daß man Verbindungen der Formel II,

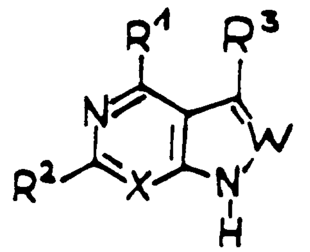

(II)

in der
X, W, $R^1$, $R^2$ und $R^3$ die in Anspruch 1 angegebene Bedeutung haben,
mit einer Verbindung der Formel III

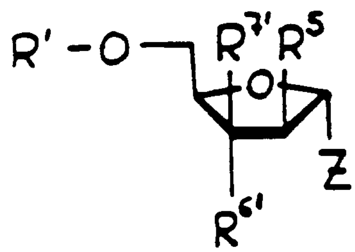

(III)

in der
$R^5$ die oben angegebene Bedeutung hat,
$R^{6'}$, $R^{7'}$ jeweils Wasserstoff oder einer der beiden Reste $R^{6'}$ und $R^{7'}$ eine Azido- oder eine durch eine Sauerstoffschutzgruppe geschützte Hydroxygruppe,
R' eine Sauerstoffschutzgruppe und
Z eine reaktive Gruppe bedeuten
zu Verbindungen der Formel IV

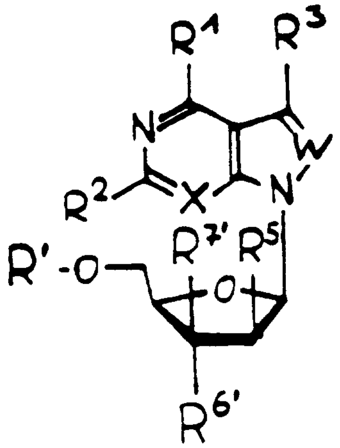

(IV)

in der
X, W, $R^1$, $R^2$, $R^3$, $R^5$, $R^{6'}$, $R^{7'}$ und R' die oben angegebene Bedeutung haben,
umsetzt und gegebenenfalls vorhandene Sauerstoffschutzgruppen abspaltet
und danach gegebenenfalls
eine so erhaltene Verbindung, in der $R^6$ oder $R^7$ eine Hydroxygruppe bedeutet, nach vorherigem selektivem Schutz der 5'-Hydroxygruppe mit einem Halogenid, Cyanid oder Azid in bekannter Weise in eine Verbindung der Formel I, in der $R^6$ oder $R^7$ Halogen, eine Cyano- oder eine Azidogruppe bedeutet, überführt oder in bekannter Weise zu einer Verbindung der Formel I, in der $R^6$ oder $R^7$

Wasserstoff bedeutet, desoxygeniert oder eine so erhaltene Verbindung der Formel I, in der $R^6$ oder $R^7$ eine Azidogruppe bedeutet, in bekannter Weise zu einer Verbindung der Formel I, in der $R^6$ oder $R^7$ eine Aminogruppe bedeutet, überführt und gewünschtenfalls anschließend Verbindungen der Formel I, in denen Y Wasserstoff bedeutet, in bekannter Weise in die Mono-, Di- oder Triphosphate überführt und gewünschtenfalls erhaltene freie Basen bzw. Säuren in die entsprechenden Salze oder erhaltene Salze in die entsprechenden freien Basen bzw. Säuren umwandelt.

**7.** Verwendung von Verbindungen gemäß den Ansprüchen 1-5 bei der DNA-Sequenzierung.

**8.** Verwendung von Verbindungen gemäß den Ansprüchen 1-5 als antivirale Mittel.

**9.** Arzneimittel, enthaltend eine Verbindung gemäß den Ansprüchen 1-5 sowie übliche Träger- und Hilfsstoffe.

**10.** Verwendung von Verbindungen gemäß den Ansprüchen 1-5 zur Herstellung von Arzneimitteln.

**Claims**

**1.** Desazapurine-nucleoside derivatives of the formula I

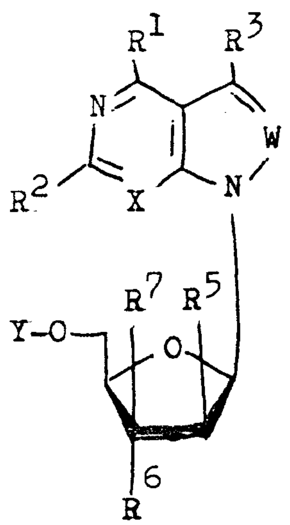

(I)

in which X represents nitrogen or a methine group, W nitrogen or the group

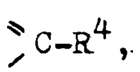

$R^1$, $R^2$, $R^3$, $R^4$, which can be the same or different, hydrogen, halogen, a lower alkyl, hydroxyl, mercapto, lower alkylthio, lower alkyloxy, aralkyl, aralkyloxy, aryloxy or an amino group possibly substituted once or twice, $R^5$ hydrogen or a hydroxyl group, $R^6$, $R^7$ in each case hydrogen or one of the two radicals $R^6$ and $R^7$ halogen, a cyano, an azido or an amino group possibly substituted once or twice, whereby one of the radicals $R^6$ and $R^7$ can also represent a hydroxyl group when X signifies a methine group and, furthermore, $R^5$ and $R^7$ can together represent a further bond between C-2' and C-3' and Y represents hydrogen, a monophosphate, diphosphate or triphosphate group, with the exception of the compounds 2',3'-didesoxytubercidin, 4,6-dichloro-1-(2-desoxy-β-D-erythropentofuranosyl)-pyrrolo[3,2-c]pyridine, 6-amino-1-(2-desoxy-β-D-erythropentofuranosyl)-pyrazolo[4,3-c]-pyridin-4-(5H)-one, 6-amino-1-(2-desoxy-β-D-erythropentofuranosyl)-1H-pyrrolo[3,2-c]pyridin-4(5H)-one, 6-amino-1-β-D-arabinofuranosyl-1H-pyrrolo[3,2-c]pyridin-4(5H)-one, 7-iodo-2',3'-didesoxy-7-desazaguanosine, 2',3'-didesoxy-2',3'-didehydro-7-desazaadenosine, 7-iodo-2',3'-didesoxy-7-desazaadenosine, 7-iodo-2',3'-didesoxy-7-desazainosine, as well as possible tautomers and salts and

nucleic acids which contain one or more compounds of the formula I as structural components.

2. Compounds according to claim 1, characterised in that W signifies the group

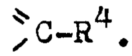

3. Compounds according to claim 1, characterised in that W signifies nitrogen.

4. Compounds according to one of claims 1 - 3, characterised in that X represents a methine group.

5. Compounds according to claim 4, characterised in that one of the radicals $R^6$ and $R^7$ signifies a hydroxyl group.

6. Process for the preparation of compounds according to claims 1 - 5, characterised in that one reacts compounds of the formula II

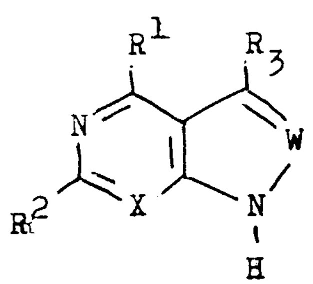

(II)

in which X, W, $R^1$, $R^2$ and $R^3$ have the meaning given in claim 1, with a compound of the formula III

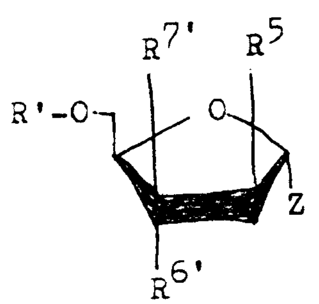

(III)

in which $R^5$ has the above-given meaning, $R^{6'}$, $R^{7'}$ are each hydrogen or one of the two radicals $R^{6'}$ and $R^{7'}$ signifies an azido or a hydroxyl group protected by an oxygen protective group, R' an oxygen protective group and Z a reactive group, to give compounds of the formula IV

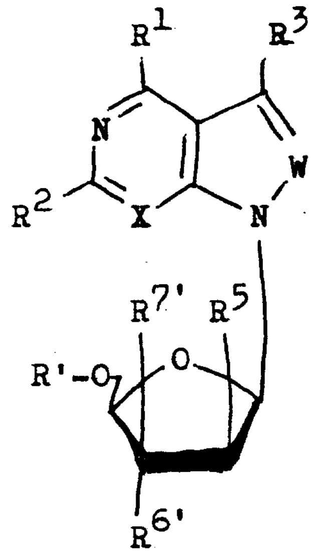

(IV)

in which X, W, $R^1$, $R^2$, $R^3$, $R^5$, $R^{6'}$, $R^{7'}$ and R' have the above-given meaning, and splits off oxygen protective groups possibly present and thereafter possibly converts a so-obtained compound, in which $R^6$ or $R^7$ signifies a hydroxyl group, after previous selective protection of the 5'-hydroxyl group with a halide, cyanide or azide, in known manner into a compound of the formula I, in which $R^6$ or $R^7$ signifies halogen, a cyano or an azido group or desoxygenates in known manner to give a compound of the formula I, in which $R^6$ or $R^7$ signifies hydrogen or converts a so-obtained compound of the formula I, in which $R^6$ or $R^7$ signifies an azido group, in known manner into a compound of the formula I, in which $R^6$ or $R^7$ signifies an amino group, and, if desired, subsequently converts in known manner compounds of the formula I, in which Y signifies hydrogen, into the mono-, di- or triphosphates and, if desired, converts free bases or acids obtained into the appropriate salts or converts salts obtained into the corresponding free bases or acids.

**7.** Use of compounds according to claims 1 - 5 for DNA sequencing.

**8.** Use of compounds according to claims 1 - 5 as antiviral agents.

**9.** Medicaments containing a compound according to claims 1 - 5, as well as usual carrier and adjuvant materials.

**10.** The use of compounds according to claims 1 - 5 for the preparation of medicaments.

## Revendications

**1.** Dérivés de désaza-purine-nucléosides de formule I

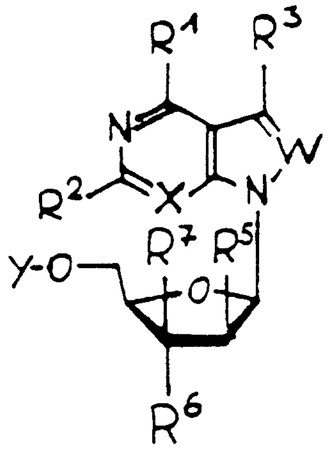

(I)

dans laquelle

X représente un atome d'azote ou un groupe méthine,

W représente un atome d'azote ou le groupe

$$>C-R^4$$

$R^1$, $R^2$, $R^3$, $R^4$, qui peuvent être identiques ou différents, représentent un atome d'hydrogène, d'halogène, un groupe alkyle inférieur, hydroxy, mercapto, thio-(alkyle inférieur), alkyloxy inférieur, aralkyle, aralkyloxy, aryloxy ou un groupe amino éventuellement une ou deux fois substitué,

$R^5$ représente un atome d'hydrogène ou un groupe hydroxy,

$R^6$, $R^7$ représentent chacun un atome d'hydrogène, ou bien les deux restes $R^6$ et $R^7$ représentent un atome d'halogène, un groupe cyano, azido ou un groupe amino éventuellement une ou deux fois substitué,

l'un des restes $R^6$ et $R^7$ pouvant également représenter un groupe hydroxy, lorsque X représente un groupe méthine,

et de plus, $R^5$ et $R^7$ peuvent représenter ensemble une autre liaison entre C-2' et C-3', et

Y représente un atome d'hydrogène, un groupe monophosphate, diphosphate ou triphosphate,

à l'exception des composés 2',3'-didésoxy-tubercidine, 4,6-dichloro-1-(2-désoxy-$\beta$-D-érythro-pentofuranosyl)-pyrrolo[3,2-c]pyridine, 6-amino-1-(2-désoxy-$\beta$-D-érythro-pentofuranosyl)pyrazolo[4,3-c]pyridin-4(5H)-one, 6-amino-1-(2-désoxy-$\beta$-D-érythro-pentofuranosyl)-1H-pyrrolo[3,2-c]pyridin-4(5H)-one, 6-amino-1-$\beta$-D-arabinofuranosyl-1H-pyrrolo[3,2-c]pyridin-4(5H)-one, 7-iodo-2',3'-didésoxy-7-désazaguanosine, 2',3'-didésoxy-2',3'-didéhydro-7-désazaadénosine, 7-iodo-2',3'-didésoxy-7-désazaadénosine, 7-iodo-2',3'-didésoxy-7-désazainosine,

ainsi que leurs tautomères et sels éventuels, et des acides nucléiques qui contiennent un ou plusieurs composés de formule I en tant que motif de base.

**2.** Composés selon la revendication 1, caractérisés en ce que W représente le groupe

$$>C-R^4.$$

**3.** Composés selon la revendication 1, caractérisés en ce que W représente l'azote.

**4.** Composés selon l'une quelconque des revendications 1-3, caractérisés en ce que X représente un groupe méthine.

**5.** Composés selon la revendication 4, caractérisés en ce que l'un des restes $R^6$ et $R^7$ représente un groupe hydroxy.

**6.** Procédé de préparation de composés selon les revendications 1-5, caractérisé en ce que l'on fait réagir des composés de formule II,

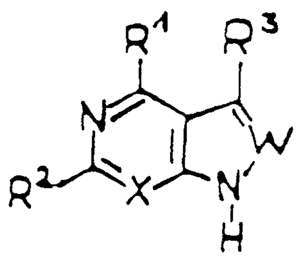

(II)

dans laquelle
X, W, $R^1$, $R^2$ et $R^3$ ont les significations mentionnées dans la revendication 1, avec un composé de formule III

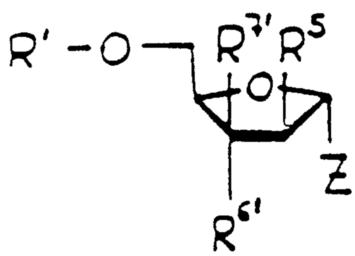

(III)

dans laquelle

| | |
|---|---|
| $R^5$ | a la signification mentionnée ci-dessus, |
| $R^{6'}$, $R^{7'}$ | représentent chacun un atome d'hydrogène, ou bien les deux restes $R^{6'}$ et $R^{7'}$ représentent un groupe azido ou un groupe hydroxy protégé par un groupe protecteur d'oxygène, |
| R' | représente un groupe protecteur d'oxygène et |
| Z | représente un groupe réactif, |

pour obtenir des composés de formule IV

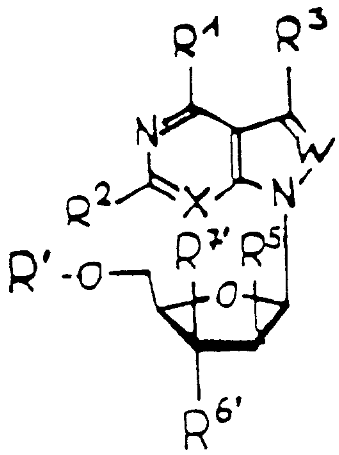

(IV)

dans laquelle X, W, $R^1$, $R^2$, $R^3$, $R^5$, $R^{6'}$, $R^{7'}$ et R' ont les significations mentionnées ci-dessus, et on sépare les groupes protecteurs d'oxygène éventuellement présents, et ensuite, on transforme éventuellement un composé ainsi obtenu, dans lequel $R^6$ ou $R^7$ représente un groupe hydroxy, après avoir protégé préalablement le groupe 5'-hydroxy au moyen d'un halogénure, cyanure ou azoture de manière connue en soi, en un composé de formule I, dans laquelle $R^6$ ou $R^7$ représente un atome d'halogène, un groupe cyano ou azido, ou de manière connue en soi, on le désoxygène en un composé de formule I dans laquelle $R^6$ ou $R^7$ représente un atome d'hydrogène, ou on transforme un composé de formule I ainsi obtenu, dans laquelle $R^6$ ou $R^7$ représente un groupe azido, de manière connue en soi, en un composé de formule I dans laquelle $R^6$ ou $R^7$ représente un groupe amino, et ensuite éventuellement, on transforme les composés de formule I, dans laquelle Y représente un atome d'hydrogène, de manière connue en soi, en le mono-, di- ou triphosphate et on transforme éventuellement les bases ou acides libres obtenus en sels correspondants ou bien on transforme éventuellement les sels obtenus en les bases ou acides libres correspondants.

**7.** Utilisation de composés selon les revendications 1-5 pour le séquençage d'ADN.

**8.** Utilisation de composés selon les revendications 1-5 en tant qu'agents antiviraux.

**9.** Médicament contenant un composé selon les revendications 1-5, ainsi que les véhicules et adjuvants usuels.

**10.** Utilisation de composés selon les revendications 1-5 pour la préparation de médicaments.